(19) Europäisches Patentamt
European Patent Office
Office européen des brevets

(11) **EP 3 177 379 B1**

(12) **EUROPÄISCHE PATENTSCHRIFT**

(45) Veröffentlichungstag und Bekanntmachung des Hinweises auf die Patenterteilung:
**12.06.2019 Patentblatt 2019/24**

(21) Anmeldenummer: **15747794.4**

(22) Anmeldetag: **04.08.2015**

(51) Int Cl.:
*B01D 3/20* (2006.01)     *B01D 3/22* (2006.01)
*B01D 3/32* (2006.01)     *B01D 3/42* (2006.01)
*C07C 67/54* (2006.01)     *C07C 45/78* (2006.01)
*C07C 45/80* (2006.01)     *C07C 45/82* (2006.01)
*C07C 51/44* (2006.01)     *C07C 51/48* (2006.01)

(86) Internationale Anmeldenummer:
**PCT/EP2015/067943**

(87) Internationale Veröffentlichungsnummer:
**WO 2016/020374 (11.02.2016 Gazette 2016/06)**

(54) **KOLONNE UND VERFAHREN ZUR THERMISCHEN BEHANDLUNG EINES FLUIDS MIT EINER RÜCKFLUSSLEITUNG**

COLUMN AND PROCESS FOR THE THERMAL TREATMENT OF A FLUID, HAVING A RETURN LINE

COLONNE ET PROCÉDÉ DE TRAITEMENT THERMIQUE D'UN FLUIDE COMPRENANT UN CONDUIT DE RETOUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.08.2014 DE 102014215437**
**05.08.2014 US 201462033157 P**

(43) Veröffentlichungstag der Anmeldung:
**14.06.2017 Patentblatt 2017/24**

(73) Patentinhaber: **BASF SE**
**67056 Ludwigshafen am Rhein (DE)**

(72) Erfinder:
• **HAMMON, Ulrich**
**68163 Mannheim (DE)**
• **WALTER, Thomas**
**67454 Haßloch (DE)**

(74) Vertreter: **BASF IP Association**
**BASF SE**
**G-FLP-C6**
**67056 Ludwigshafen (DE)**

(56) Entgegenhaltungen:
EP-A1- 0 009 545     EP-A1- 2 460 577
CA-A1- 2 017 980     DE-A1- 19 924 532
US-A- 2 141 829

**Beschreibung**

Die vorliegende Erfindung betrifft eine Kolonne zur thermischen Behandlung eines Fluids. Die Kolonne weist einen zylindrischen, vertikal ausgerichteten Kolonnenkörper, der einen Kolonnenhohlraum bildet, und einen in dem Kolonnenhohlraum angeordneten Stoffaustauschboden auf, der eine Auffangfläche bildet. Bei der Kolonne handelt es sich insbesondere um eine Trennkolonne. Des Weiteren betrifft die Erfindung ein thermisches Trennverfahren zwischen wenigstens einem in einer Kolonne aufsteigenden Gas und wenigstens einer in der Kolonne absteigenden Flüssigkeit.

In Trennkolonnen werden vielfach gasförmige (aufsteigend) und flüssige (absteigend) Stoffströme im Gegenstrom geführt, wobei wenigstens einer der Stoffströme insbesondere ein (Meth)acrylmonomer enthält. Infolge der zwischen den Stoffströmen bestehenden Ungleichgewichte findet ein Wärme- und Stoffaustausch statt, der letztlich die in der Trennkolonne gewünschte Abtrennung (bzw. Auftrennung) bedingt. In dieser Schrift sollen solche Trennverfahren als thermische Trennverfahren bezeichnet werden.

Beispiele für und damit Element der in dieser Schrift verwendeten Ausdrucksweise "thermische Trennverfahren" sind die fraktionierende Kondensation (vgl. z.B. DE 19924532 A1, DE 10243625 A1 und WO 2008/090190 A1) und die Rektifikation (bei beiden wird aufsteigende Dampfphase im Gegenstrom zu absteigender Flüssigphase geführt; die Trennwirkung beruht darauf, dass die Dampfzusammensetzung im Gleichgewicht von der Flüssigzusammensetzung verschieden ist), die Absorption (wenigstens ein aufsteigendes Gas wird zu wenigstens einer absteigenden Flüssigkeit im Gegenstrom geführt; die Trennwirkung beruht auf der unterschiedlichen Löslichkeit der Gasbestandteile in der Flüssigkeit) und die Desorption (der Umkehrprozess zur Absorption; das in der Flüssigphase gelöste Gas wird durch Partialdruckerniedrigung abgetrennt; erfolgt die Partialdruckerniedrigung des in der Flüssigphase Gelösten wenigstens teilweise dadurch, dass ein Trägergas durch die Flüssigphase geleitet wird, bezeichnet man dieses thermische Trennverfahren auch als Strippung; alternativ oder auch zusätzlich (zeitgleich als Kombination) kann die Partialdruckerniedrigung durch eine Absenkung des Arbeitsdruckes bewirkt werden).

Beispielsweise kann die Abtrennung von (Meth)acrylsäure bzw. (Meth)acrolein aus dem Produktgasgemisch der katalytischen Gasphasenoxidation so durchgeführt werden, dass die (Meth)acrylsäure bzw. das (Meth)acrolein durch Absorption in ein Lösungsmittel (z.B. Wasser oder ein organisches Lösungsmittel) oder durch fraktionierende Kondensation des Produktgasgemisches zunächst grundabgetrennt und das dabei anfallende Absorbat bzw. Kondensat nachfolgend unter Erhalt von mehr oder weniger reiner (Meth)acrylsäure bzw. (Meth)acrolein weiter aufgetrennt wird (vgl. z.B. DE-10332758 A1, DE 10243625 A1, WO 2008/090190 A1, DE 10336386 A1, DE 19924532 A1, DE 19924533 A1, DE 102010001228 A1, WO 2004/035514 A1, EP 1125912 A2, EP 982289 A2, EP 982287 A1 und DE 10218419 A1).

Die Schreibweise (Meth)acrylmonomere steht in dieser Schrift verkürzend für "Acrylmonomere und/oder Methacrylmonomere".

Der Begriff Acrylmonomere steht in dieser Schrift verkürzend für "Acrolein, Acrylsäure und/oder Ester der Acrylsäure".

Der Begriff Methacrylmonomere steht in dieser Schrift verkürzend für "Methacrolein, Methacrylsäure und/oder Ester der Methacrylsäure".

Im Besonderen sollen die in dieser Schrift angesprochenen (Meth)acrylmonomere die nachfolgenden (Meth)acrylsäureester umfassen: Hydroxyethylacrylat, Hydroxyethylmethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, iso-Butylmethacrylat, n-Butylmethacrylat, tert.-Butylacrylat, tert.-Butylmethacrylat, Ethylacrylat, Ethylmethacrylat, 2-Ethylhexylacrylat, 2-Ethylhexylmethacrylat, N,N-Dimethylaminoethylacrylat und N,N-Dimethylaminoethylmethacrylat.

(Meth)acrylmonomere sind wichtige Ausgangsverbindungen zur Herstellung von Polymerisaten, die z.B. als Klebstoffe oder als Wasser super absorbierende Materialien in Hygieneartikeln Verwendung finden.

Großtechnisch werden (Meth)acrolein und (Meth)acrylsäure vorwiegend durch katalytische Gasphasenoxidation geeigneter $C_3$-/$C_4$-Vorläuferverbindungen (oder von Vorläuferverbindungen derselben) hergestellt. Im Fall von Acrolein und Acrylsäure werden als solche Vorläuferverbindungen bevorzugt Propen und Propan verwendet. Im Fall der Methacrylsäure und des Methacroleins sind iso-Buten und iso-Butan die bevorzugten Vorläuferverbindungen.

Neben Propen, Propan, iso-Buten und iso-Butan eignen sich als Ausgangsstoffe jedoch auch andere 3 bzw. 4 Kohlenstoffatome enthaltende Verbindungen wie z.B. iso-Butanol, n-Propanol oder Vorläuferverbindungen derselben wie z.B. der Methylether von iso-Butanol. Acrylsäure kann auch durch gasphasenkatalytische Oxidation von Acrolein erzeugt werden. Methacrylsäure kann auch durch gasphasenkatalytische Oxidation von Methacrolein erzeugt werden.

Im Rahmen solcher Herstellverfahren werden normalerweise Produktgasgemische erhalten, aus welchen die (Meth)acrylsäure bzw. das (Meth)acrolein abgetrennt werden muss.

Ester der (Meth)acrylsäure sind z.B. durch direkte Umsetzung von (Meth)acrylsäure und/oder (Meth)acrolein mit den entsprechenden Alkoholen erhältlich. Allerdings fallen auch in diesem Fall zunächst Produktgemische an, aus denen die (Meth)acrylsäureester abgetrennt werden müssen.

Die Trennkolonnen, in denen diese Trennverfahren durchgeführt werden, enthalten trennwirksame Einbauten. Diese verfolgen bei den thermischen Trennverfahren den Zweck, die Oberfläche für den die Auftrennung in der Trennkolonne bewirkenden Wärme- und Stoffaustausch ("die Austauschfläche") zu erhöhen.

Als solche Einbauten kommen z.B. Packungen, Füllkörper und/oder Böden, die auch als Stoffaustauschböden bezeichnet werden, in Betracht. Häufig werden als Trennkolonnen solche verwendet, die wenigstens als einen Teil der trennwirksamen Einbauten wenigstens eine Abfolge von Stoffaustauschböden enthalten.

Stoffaustauschböden verfolgen den Zweck, in der Trennkolonne in Form von auf ihnen sich ausbildenden Flüssigkeitsschichten Gebiete mit im Wesentlichen geschlossenen flüssigen Phasen zur Verfügung zu stellen. Die Oberfläche des in der Flüssigkeitsschicht aufsteigenden und sich dabei in der flüssigen Phase verteilenden Dampf- bzw. Gasstroms ist dann die maßgebliche Austauschfläche.

Unter einer Abfolge von Stoffaustauschböden wird dabei eine Aufeinanderfolge (ein Nacheinander) von wenigstens zwei in der Trennkolonne übereinander angeordneten, im Regelfall baugleichen (d.h., identischen), Stoffaustauschböden verstanden. Anwendungstechnisch vorteilhaft ist der lichte Abstand zwischen zwei in einer solchen Serie (Reihe) von Stoffaustauschböden unmittelbar aufeinanderfolgenden Stoffaustauschböden einheitlich gestaltet (d.h., die Stoffaustauschböden sind in der Trennkolonne äquidistant übereinander angeordnet).

Die einfachste Ausführungsform eines Stoffaustauschbodens ist der sogenannte Regensiebboden. Dabei handelt es sich um eine Platte bzw. um zu einer Platte zusammengefügte Plattensegmente, die für die aufsteigende Gasbzw. Dampfphase (die Begriffe "gasförmig" und "dampfförmig" werden in dieser Schrift synonym verwendet) über die Platte verteilte und im Wesentlichen plane Durchtrittsöffnungen, z.B. runde Löcher und/oder Schlitze, aufweist (vgl. z.B. DE 10230219 A1, EP 1279429 A1, US-A 3988213 und EP 1029573 A1). Darüber hinausgehende Öffnungen (z.B. wenigstens einen Ablaufschacht (wenigstens ein Ablaufsegment)) weisen Regensiebböden nicht auf. Durch diese Abwesenheit von Ablaufschächten müssen sich sowohl das in der Trennkolonne aufsteigende Gas (der in der Trennkolonne aufsteigende Dampf) als auch die in der Trennkolonne absteigende Flüssigkeit entgegengesetzt strömend im zeitlichen Wechsel durch die (gleichen) Durchtrittsöffnungen (durch die offenen Querschnitte der Durchtrittstellen) bewegen. Man spricht auch vom "dual-flow" von aufsteigendem Gas und absteigender Flüssigkeit durch die Durchtrittsöffnungen, weshalb in der Literatur für solche Stoffaustauschböden häufig auch der Begriff "Dual-Flow-Böden" verwendet wird.

Der Querschnitt der Durchtrittsöffnungen eines Dual-Flow-Bodens wird in an sich bekannter Weise seiner Belastung angepasst. Ist er zu klein, strömt das aufsteigende Gas mit so hoher Geschwindigkeit durch die Durchtrittsöffnungen, dass die in der Trennkolonne absteigende Flüssigkeit im Wesentlichen ohne Trennwirkung mitgerissen wird. Ist der Querschnitt der Durchtrittsöffnungen zu groß, bewegen sich aufsteigendes Gas und absteigende Flüssigkeit im Wesentlichen ohne Austausch aneinander vorbei und der Stoffaustauschboden läuft Gefahr trocken zu laufen.

D.h., der trennwirksame Arbeitsbereich eines Regensiebbodens (Dual-Flow-Boden) weist zwei Grenzen auf. Eine minimale Grenzgeschwindigkeit des aufsteigenden Gases muss gegeben sein, damit auf dem Regensiebboden eine gewisse Flüssigkeitsschicht gehalten wird, um ein trennwirksames Arbeiten des Regensiebbodens zu ermöglichen. Die obere Grenze der Geschwindigkeit des aufsteigenden Gases ist durch den Flutpunkt festgelegt, wenn die Gasgeschwindigkeit zum Stau der Flüssigkeit auf dem Regensiebboden führt und ihr Durchregnen verhindert wird.

Die Längstausdehnung der Durchtrittsöffnungen eines technischen Dual-Flow-Bodens (= längste direkte Verbindungslinie zweier auf der Umrisslinie des Querschnitts der Durchtrittsöffnung liegender Punkte) beträgt in typischer Weise 10 bis 80 mm (vgl. z.B. DE 10156988 A1). Normalerweise sind die Durchtrittsöffnungen innerhalb eines Regensiebbodens identisch (d.h., sie weisen alle die gleiche geometrische Form und den gleichen Querschnitt (die gleiche Querschnittsfläche) auf). Anwendungstechnisch zweckmäßig handelt es sich bei ihren Querschnittsflächen um Kreise. D.h., bevorzugte Durchtrittsöffnungen von Regensiebböden sind kreisförmige Bohrungen. Die Relativanordnung der Durchtrittsöffnungen eines Regensiebbodens folgt vorteilhaft einer strengen Dreiecksteilung (vgl. z.B. DE 10230219 A1). Selbstverständlich können die Durchtrittsöffnungen innerhalb ein und desselben Regensiebbodens auch unterschiedlich gestaltet sein (über den Regensiebboden variieren).

Anwendungstechnisch vorteilhaft umfasst eine Abfolge von Regensiebböden in einer Trennkolonne baugleiche (identische) Regensiebböden, die vorzugsweise äquidistant übereinander angeordnet sind.

Gemäß der DE 10156988 A1 können aber auch Abfolgen von Regensiebböden in Trennkolonnen zur Anwendung kommen, deren Querschnitt (bevorzugt kreisförmig) innerhalb eines Dual-Flow-Bodens zwar einheitlich gestaltet ist, innerhalb der Abfolge jedoch variiert (z.B. von unten nach oben abnimmt).

In der Regel schließt jeder Dual-Flow-Boden einer entsprechenden Bodenabfolge mit der Wand der Trennkolonne bündig ab. Es gibt aber auch Ausführungsvarianten, bei denen zwischen Kolonnenwand und Boden ein Zwischenraum besteht, der nur teilweise durch Brücken unterbrochen ist. Neben den eigentlichen Durchtrittsöffnungen weist ein Regensiebboden üblicherweise allenfalls noch Öffnungen auf, die der Befestigung des Bodens auf Auflageringen oder ähnlichem dienen (vgl. z.B. DE 10159823 A1).

Im normalen Arbeitsbereich einer Abfolge von Regensiebböden regnet die in der Trennkolonne absteigende Flüssigkeit in Tropfen von Dual-Flow-Boden zu Dual-Flow-Boden, d.h., die zwischen den Dual-Flow-Böden aufsteigende Gasphase wird von einer zerteilten Flüssigkeitsphase durchsetzt. Die auf dem jeweils unteren Regensiebboden auftreffenden Tropfen werden beim Auftreffen teilweise versprüht. Der durch die Durchtrittsöffnungen strömende Gasstrom sprudelt durch die auf der Oberfläche des Bodens gebildete Flüssigkeitsschicht, wobei ein intensiver Stoff- und Wärmeaustausch zwischen der Flüssigkeit und dem Gas stattfindet.

Je nach Flüssigkeits- und Gasbelastung neigen Regensiebböden bei Kolonnendurchmesser von >2 m dazu, dass sich geringe Ungleichverteilungen von Flüssigkeiten aufschaukeln können und so der Flüssigkeitsholdup eines Bodens großflächig schwankt bzw. sich eine umlaufende Welle ausbilden kann, was zum einen die mechanische Stabilität des Kolonnenkörpers negativ beeinflussen kann und zum anderen die Trennwirkung vermindert, da die Flüssigkeitsverteilung unter diesen Bedingungen dann zeitlich und örtlich stark unterschiedlich ist. Zur Vermeiden solcher Instationaritäten hat es sich deshalb als vorteilhaft erwiesen Strömungsbrecher in Form von senkrecht stehenden Blechen auf dem Bodenquerschnitt zu verteilen, die ein Aufschaukeln der Flüssigkeit innerhalb des Kolonnenkörpers verhindern oder zumindest stark reduzieren. Die Höhe der Bleche sollte dabei in etwa der Höhe der sich ausbildenden Flüssigkeitssprudelschicht entsprechen. Diese beträgt bei üblichen Belastungen ca. 20 cm.

Der Querschnitt einer Trennkolonne ist in der Regel kreisförmig. Dies trifft in entsprechender Weise auf die zugehörigen Stoffaustauschböden zu.

Für die Zwecke dieser Schrift verwendbare Dual-Flow-Böden sind z.B. in Technische Fortschrittsberichte, Bd. 61, Grundlagen der Dimensionierung von Kolonnenböden, Seite 198 bis 211, Verlag Theodor Steinkopf, Dresden (1967) und in der DE 10230219 A1 beschrieben.

Von der vorstehend beschriebenen Abfolge von Regensiebböden, die Stoffaustauschböden ohne Zwangsführung der auf den Boden absteigenden Flüssigkeit auf dem Boden umfasst, werden Abfolgen von Stoffaustauschböden mit einer solchen Flüssigkeitszwangsführung unterschieden.

Diese Stoffaustauschböden sind dadurch gekennzeichnet, dass sie neben den bereits beschriebenen Durchtrittsöffnungen zusätzlich wenigstens einen Ablaufschacht aufweisen. Dabei handelt es sich um wenigstens eine im Stoffaustauschboden befindliche Ablauföffnung, der die auf den Stoffaustauschboden abgestiegene Flüssigkeit (z.B. über ein Ablaufwehr (dieses kann in einfachster Ausführungsform eine Verlängerung der Ablauföffnung mit einem Hals (einem Kamin; im Fall einer kreisförmigen Ablauföffnung einer Röhre) nach oben sein)) zufließt, und die in einen zum in der Abfolge darunter liegenden Stoffaustauschboden zulaufenden Schacht ausläuft, der in der Regel zu einer in Kolonnenlängsrichtung weisenden Achse zentralsymmetrisch ausgebildet ist. Der Querschnitt des Schachts kann entlang dieser Achse variieren (sich z.B. verjüngen) oder auch konstant sein.

Durch den wenigstens einen Ablaufschacht des Stoffaustauschbodens kann innerhalb einer Abfolge derartiger Stoffaustauschböden die von einem höher gelegenen Stoffaustauschboden absteigende Flüssigkeit unabhängig vom nach wie vor durch die Durchtrittsöffnungen dieses Stoffaustauschbodens aufsteigenden Gas bzw. Dampf als wenigstens ein Zulauf an Flüssigkeit auf den nächst tiefer gelegenen Stoffaustauschboden der Abfolge absteigen.

Wesentliche Grundlage für diese Auftrennung der Strömungswege von absteigender Flüssigkeit und aufsteigendem Gas ist der hydraulische Verschluss (der Flüssigkeitsverschluss oder auch Schachtverschluss) des jeweiligen Ablaufschachtes für das aufsteigende Gas (ein Ablaufschacht darf für das aufsteigende Gas keinen Bypass an den Durchtrittsöffnungen vorbei bilden; der Gasstrom (der Dampfstrom) darf nicht durch einen Ablaufschacht an den Durchtrittsöffnungen vorbei aufsteigen).

Ein solcher hydraulischer Verschluss kann z.B. dadurch erreicht werden, dass man den Ablaufschacht so weit nach unten zieht (so weit nach unten auslaufen lässt), dass er tief genug in die auf dem nächst tiefer gelegenen Stoffaustauschboden der Abfolge befindliche Flüssigkeitsschicht eintaucht (ein solcher Verschluss wird in dieser Schrift auch als "statischer Verschluss" bezeichnet). Der hierfür notwendige Flüssigkeitsstand kann auf dem tiefer gelegenen Stoffaustauschboden z.B. durch die Höhe entsprechender Ablaufwehre gewährleistet werden.

Eine derartige Ausführung hat jedoch den Nachteil, dass der Bereich des tiefer gelegenen Stoffaustauschbodens, der sich unmittelbar unterhalb des Auslaufquerschnitts eines Ablaufschachtes des darüber befindlichen Stoffaustauschbodens (die sogenannte Zulauffläche) befindet, keine Durchtrittsöffnungen für das aufsteigende Gas aufweisen kann und so nicht für den Stoff- und Wärmeaustausch zwischen der auf dem tiefer gelegenen Stoffaustauschboden ausgebildeten Flüssigkeitsschicht und dem aufsteigenden Gas zur Verfügung steht.

Bei einer alternativen Ausführungsform ist das untere Auslaufende des Ablaufschachtes so weit hochgezogen, dass es nicht mehr in die auf dem darunter liegenden Stoffaustauschboden befindliche Flüssigkeitsschicht eintaucht. In diesem Fall verbleibt zwischen dem unteren Ende des wenigstens einen Ablaufschachts und dem Stoffaustauschboden, auf den der Ablaufschacht zuläuft, ein ausreichend großer Zwischenraum, in dem sich eine Sprudelschicht ausbilden und ein Stoff- und Wärmeaustausch zwischen einer (auf dem unteren Stoffaustauschboden) auflaufenden Flüssigkeitsschicht und einem (durch diesen Boden) aufsteigenden Gas stattfinden kann. D.h., in diesem Fall kann auch die "Zulauffläche" des wenigstens einen Ablaufschachtes auf dem darunter befindlichen Stoffaustauschboden Durchtrittsöffnungen aufweisen und so die verfügbare Austauschfläche des Stoffaustauschbodens, und damit seine Trennwirkung vergrößert werden.

Ein statischer Flüssigkeitsverschluss des Ablaufschachtes kann in diesem Fall z.B. mit Hilfe einer unter dem Auslaufende des Ablaufschachtes angebrachten Auffangtasse bewirkt werden. Anwendungstechnisch zweckmäßig wird in diesem Fall die Mantelwand der Auffangtasse so weit hochgezogen, dass das Auslaufende des Ablaufschachts in die Auffangtasse eintaucht (es ist auch möglich, die Unterkante des Ablaufschachts an der Oberkante der Auffangtasse enden zu lassen). Beim Betrieb der Kolonne sammelt sich in der Auffangtasse die durch den Ablaufschacht herabströmende Flüssigkeit so lange, bis diese über die Oberkante der Mantelwand der Auffangtasse abfließt. Die Unterkante des Ablaufschachts taucht in die in der Auffangtasse befindliche Flüssigkeit ein und die Auffangtasse bildet einen siphonartigen Flüssigkeitsverschluss des Ablaufschachtes.

Alternativ kann ein hochgezogener Ablaufschacht auch dynamisch verschlossen werden. Hierzu kann der Ablaufschacht z.B. an seinem unteren Ende mit einem Boden verschlossen werden, der mit Austrittsöffnungen versehen ist, die so dimensioniert sind, dass die Flüssigkeit im Ablaufschacht aufgestaut und das Eindringen von Gas verhindert wird (vgl. z.B. EP 0882481 A1 und DE 10257915 A1). Der Schachtverschluss wird in diesem Fall dynamisch durch den Druckverlust, der an den Austrittsöffnungen entsteht, hergestellt. D.h., beim statischen Verschluss erfolgt der Verschluss des Ablaufschachtes dadurch, dass dessen Auslaufende in gestaute Flüssigkeit eintaucht, und beim dynamischen Verschluss bewirken konstruktive Merkmale am Auslaufende des Ablaufschachts, dass die austretende (auslaufende) Flüssigkeit einen Druckverlust erleidet, der im Ablaufschacht einen Rückstau der in selbigem absteigenden Flüssigkeit bewirkt, welcher den Verschluss bedingt. Im einfachsten Fall kann ein solcher Druckverlust dadurch verursacht werden, dass man den Querschnitt der Austrittsöffnung des Ablaufschachts im Vergleich zum mittleren Querschnitt des Schachts klein wählt.

Für ein trennwirksames Arbeiten einer Abfolge von derartigen Stoffaustauschböden ist die Ausführung des wenigstens einen Ablaufschachtes relevant. Einerseits muss der Querschnitt des wenigstens einen Ablaufschachts hinreichend groß gewählt werden (in der Regel ist die entsprechende Querschnittsfläche größer als die Querschnittsfläche einer Durchtrittsöffnung), damit die Flüssigkeit auch bei der maximalen Belastung der Trennkolonne mit selbiger noch sicher durch den wenigstens einen Ablaufschacht absteigen kann und nicht bis auf den darüber liegenden Boden zurückstaut. Auf der anderen Seite muss sichergestellt werden, dass auch bei minimaler Flüssigkeitsbelastung der hydraulische Verschluss des wenigstens einen Ablaufschachtes noch besteht.

Bei geringer Gasbelastung besteht ebenfalls die Gefahr eines Durchregnens von Flüssigkeit durch die Durchtrittsöffnungen. Darüber hinaus muss sich die Flüssigkeit in einem Ablaufschacht so weit aufstauen können, bis das Gewicht der gestauten Flüssigkeitssäule ausreicht, um die Flüssigkeit in den Gasraum unterhalb des zum Ablaufschachts gehörigen Stoffaustauschbodens zu befördern. Diese Rückstauhöhe bestimmt die erforderliche Mindestlänge des Ablaufschachts und bestimmt so den in einer Abfolge entsprechender Stoffaustauschböden erforderlichen Bodenabstand mit. Wesentlicher Mitbestimmungsfaktor für vorstehende Rückstauhöhe (Rückstaulänge) ist der Druckverlust $\Delta P$ eines Stoffaustauschbodens. Diesen Druckverlust erleidet das aufsteigende Gas beim Durchströmen der Durchtrittsöffnungen sowie der "hydrostatischen" Höhe der Sprudelschicht auf dem Stoffaustauschboden. Er ist dafür verantwortlich, dass der Druck in der Gasphase einer Abfolge solcher Stoffaustauschböden von oben nach unten zunimmt. Für den "hydrostatischen" Druck $h_p$ der im Ablaufschacht gestauten Flüssigkeit eines Stoffaustauschbodens muss daher wenigstens die Bedingung $h_p > \Delta P$ des Stoffaustauschbodens erfüllt sein. Diese Zusammenhänge sind dem Fachmann z.B. aus der EP 1704906 A1 ebenso bekannt, wie die Möglichkeit, mit einem Zulaufwehr auf dem tiefer liegenden Stoffaustauschboden sicherzustellen, dass bei statischem Verschluss des Ablaufschachtes des oberhalb gelegenen Stoffaustauschbodens in der Flüssigkeitsschicht auf dem tiefer liegenden Stoffaustauschboden, der Schachtverschluss auch bei geringer Belastung mit absteigender Flüssigkeit noch besteht. Allerdings erhöht die Mitverwendung eines Zulaufwehrs die Rückstauhöhe, die im Ablaufschacht erforderlich ist, um die in selbigem gestaute Flüssigkeit auf den tiefer gelegenen Stoffaustauschboden zu drücken. Insgesamt ermöglicht das Element des Ablaufschachts eine Verbreiterung des trennwirksamen Arbeitsbereichs im Vergleich zum Regensiebboden. Eine günstige Ablaufgeschwindigkeit der im Ablaufschacht gestauten Flüssigkeit aus dem Ablaufschacht heraus beträgt beim erfindungsgemäßen Verfahren z.B. 1,2 m/s.

Zusätzlich ermöglicht es eine Zwangsführung der auf einen Stoffaustauschboden absteigenden Flüssigkeit auf diesem Boden.

Weist z. B. nur eine Hälfte eines (vorzugsweise kreisförmigen) Stoffaustauschbodens wenigstens einen Ablaufschacht auf (d.h., alle Ablauföffnungen befinden sich mit ihrem vollen Umfang innerhalb des entsprechenden Kreissegments) und sind in einer Abfolge von wenigstens zwei baugleichen derartigen Stoffaustauschböden die Stoffaustauschböden in einer Trennkolonne so übereinander angeordnet, dass zwei von oben nach unten aufeinanderfolgende Stoffaustauschböden in der Trennkolonne jeweils um 180° um die Kolonnenlängsachse gegeneinander verdreht (gedreht) angebracht sind, so dass sich ihre Ablaufschächte auf einander gegenüber liegenden Seiten (in einander gegenüberliegenden Hälften) der Trennkolonne befinden, so muss sich die von einem oberen Stoffaustauschboden durch seinen wenigstens einen Ablaufschacht auf den darunter angebrachten Stoffaustauschboden absteigende Flüssigkeit auf diesem unteren Stoffaustauschboden über den unteren Stoffaustauschboden betrachtet von der wenigstens einen Zulauffläche des wenigstens einen Ablaufschachts des oberen (des darüber angebrachten) Stoffaustauschbodens (von dem wenigstens einen Zulauf durch den wenigstens einen Ablaufschacht des oberen Stoffaustauschbodens) in notwendiger Weise (d. h., gezwungenermaßen) zu dem wenigstens einen Ablaufschacht dieses unteren Stoffaustauschbodens strömen. D. h., die vom oberen auf den unteren Boden absteigende Flüssigkeit wird zwangsweise quer über den Boden von dem wenigstens einen Zulauf zu dem wenigstens einen Ablauf geführt.

Eine solche Flüssigkeitsführung auf einem Stoffaustauschboden innerhalb einer Abfolge von baugleichen Stoffaustauschböden soll in dieser Schrift als eine Querströmung, die Abfolge von solchen baugleichen Stoffaustauschböden als eine Abfolge von baugleichen Querstrom-Stoffaustauschböden und der einzelne Stoffaustauschboden innerhalb der Abfolge als Querstrom-Stoffaustauschboden bezeichnet werden.

Im einfachsten Fall ist der Querstrom-Stoffaustauschboden ein Querstrom-Siebboden. Abgesehen von dem wenigstens einen Ablaufschacht weist er Durchtrittsöffnungen für das in einer Trennkolonne aufsteigende Gas auf, für deren Ausgestaltung grundsätzlich alle beim Regensiebboden angesprochenen Ausführungsformen in Betracht kommen. Vorzugsweise weist ein Querstrom-Siebboden als Durchtrittsöffnungen ebenfalls kreisförmige Bohrungen auf, die anwendungstechnisch vorteilhaft ebenfalls einen einheitlichen Radius aufweisen. Wie bereits erwähnt, ermöglicht es der wenigstens eine Ablaufschacht, dass die in einer Trennkolonne absteigende Flüssigkeit in einer Abfolge von Querstrom-Siebböden unabhängig vom Strömungsweg des in der Abfolge aufsteigenden Dampfes (durch die Durchtrittsöffnungen hindurch) von einem höher gelegenen Querstrom-Siebboden auf den nächst tiefer gelegenen Querstrom-Siebboden absteigen kann. Auf dem tiefer gelegenen Boden fließt die Flüssigkeit im Querstrom von dem durch den wenigstens einen Ablauf des höher gelegenen Querstrom-Siebbodens gebildeten wenigstens einen Zulauf des tiefer gelegenen Bodens zu dem wenigstens einen Ablaufschacht (zu dem wenigstens einen Ablauf) des tiefer gelegenen Bodens, wobei z.B. die Höhe von wenigstens einem Ablaufwehr, über das die Flüssigkeit dem wenigstens einen Ablaufschacht zufließen kann, die gewünschte Flüssigkeitshöhe auf dem tiefer gelegenen Querstrom-Siebboden mit gewährleistet. Zusätzlich wird die Flüssigkeit durch den Staudruck des in der Trennkolonne aufsteigenden Dampfes auf dem Querstrom-Siebboden gehalten. Sinkt die Dampfbelastung eines Querstrom-Siebbodens jedoch unter einen Mindestwert, kann es zum Durchregnen der Flüssigkeit durch die Durchtrittsöffnungen kommen, was die Trennwirkung des Querstrom-Siebbodens mindert und/oder zum Trockenlaufen des Querstrom-Siebbodens führt.

Dieser Gefahr des Trockenlaufens kann dadurch entgegengewirkt werden, dass die Ablauföffnung des wenigstens einen Ablaufschachts ablaufbewehrt ist und die jeweilige Durchtrittsöffnung mit einem Hals (einem Kamin; im Fall einer kreisförmigen Durchtrittsöffnung einer Röhre) nach oben verlängert wird.

Über dem Halsende sind normalerweise Dampfumlenkhauben (Glocken, umgedrehte Tassen) angebracht (diese können im einfachsten Fall mit dem Hals (z.B. vorne und hinten) verschraubt aufsitzen und werden praktisch über den Hals gestülpt), die in die auf dem Boden aufgestaute Flüssigkeit eintauchen. Der durch die jeweilige Durchtrittsöffnung aufsteigende Dampf strömt zunächst durch deren Hals in die zugehörige Haube, in welcher er umgeleitet wird, um anschließend, im Unterschied zum Querstrom-Siebboden, parallel zur Bodenfläche aus der Haube in die auf selbiger gestaute Flüssigkeit zu strömen (eine solche "Parallelausströmung" ist bei erfindungsgemäßen Verfahren in der Regel insofern günstig, als sie in unerwünschter Weise gebildete Polymerisatpartikel "wegzublasen" und dadurch einen Selbstreinigungseffekt zu bewirken vermag). Die aus benachbarten, über den Böden vorzugsweise äquidistant verteilt angeordneten, Hauben austretenden Gasströme (Dampfströme) wirbeln die auf dem Boden gestaute Flüssigkeit auf und bilden in selbiger eine Sprudelschicht aus, in der der Stoff- und Wärmeaustausch stattfindet. Solche Querstrom-Stoffaustauschböden werden auch als Querstrom-Glockenböden bzw. Querstrom-Haubenböden bezeichnet. Da sie auch bei geringer Belastung mit aufsteigendem Gas (Dampf) gestaute Flüssigkeit aufweisen und so keine Gefahr laufen, trocken zu laufen, werden sie auch als hydraulisch abgedichtete Querstromböden bezeichnet. Im Vergleich zu Querstrom-Siebböden bedürfen sie üblicherweise höherer Investitionskosten und bedingen höhere Druckverluste des durch sie hindurch aufsteigenden Gases. Die wie beschrieben ausgeführte (ausgestaltete) Durchtrittsöffnung dieser Böden wird im Unterschied zur einfachen Siebdurchtrittsöffnung eines Siebbodens auch als Glockendurchtrittsöffnung bzw. Haubendurchtrittsöffnung bezeichnet.

Das wichtigste Bauelement des Querstrom-Glockenbodens ist die Glocke (vgl. z. B. DE 10243625 A1 und Chemie-Ing.-Techn. 45. Jahrg. 1973/Nr. 9 + 10, S. 617 bis 620). Je nach Gestalt und Anordnung der Glocken (Dampfumlenkhauben, Hauben) unterscheidet man Querstrom-Glockenböden z. B. in Querstrom-Rundglockenböden (die Querschnitte von Durchtrittsöffnung, Kamin (Hals) und Glocke (Dampfumlenkhaube) sind rund (z. B. der Zylinderglockenboden oder der Flachglockenboden), Tunnel-Querstromböden (die Querschnitte von Durchtrittsöffnung, Kamin und Glocke (Haube) sind rechteckig, die Durchtrittstellen mit ihren Glocken sind innerhalb von nebeneinander angeordneten Reihen hintereinander angeordnet, wobei die längere Rechteckkante parallel zur Querstromrichtung der Flüssigkeit ausgerichtet ist) und Querstrom-Thormann®böden (die Querschnitte von Durchtrittsöffnung, Kamin und Glocke (Haube) sind rechteckig, die Durchtrittstellen mit ihren Glocken sind innerhalb von nebeneinander angeordneten Reihen hintereinander angeordnet, wobei die längere Rechteckkante senkrecht zur Querstromrichtung der Flüssigkeit ausgerichtet ist). Querstrom-Thormannböden sind z. B. in der DE 19924532 A1 und in der DE 10243625 A1 und dem in diesen beiden Schriften gewürdigten Stand der Technik beschrieben.

Der Glockenrand kann bei Querstrom-Glockenböden sehr verschiedene Formen aufweisen (vgl. DE 10243625 A1 und Chemie-Ing. Techn. 45. Jahrg. 1973/Nr. 9 + 10, S. 617 bis 620). Abbildung 3 aus Chemie-Ing. Techn. 45 Jahr. 1973/Nr. 9 + 10, S. 618  zeigt einige Beispiele für den gezackten und den geschlitzten Rand. Die Zacken und Schlitze sind üblicherweise so geformt, dass sich der aus der Glocke heraus in die auf dem Stoffaustauschboden aufgestaute Flüssigkeit eintretende Dampf möglichst leicht in eine große Zahl von Blasen oder Dampfstrahlen auflöst. Vorgenannte Abbildung 3 sowie verschiedene Figuren der DE 10243625 A1 zeigen außerdem beispielhafte Ausführungsformen von

EP 3 177 379 B1

Glockenrändern, die eine sägezahnartige Struktur aufweisen, deren Zähne zusätzlich mit Leitflügeln (Leitflächen) ausgestattet sind ("aufgebogene Schlitze"). Die Leitflügel sollen dem aus den aufgebogenen sägezahnartigen Schlitzen austretenden Gasstrom (Dampfstrom) eine tangentiale Austrittsrichtung aufzwingen (den Gasaustritt in die Flüssigkeit in eine schräge Richtung leiten) wodurch die umgebende Flüssigkeit einen gerichteten Bewegungsimpuls erhält, was im Zusammenwirken mit der Anordnung der Glocken (Dampfumlenkhauben) zu einer gerichteten Flüssigkeitsströmung auf dem Querstrom-Glockenboden führen kann, die sich der über den Stoffaustauschboden betrachtet einstellenden Querströmung überlagert (häufig werden derartige aufgebogene Schlitze auch als Treibschlitze bezeichnet). Beispielsweise fließt in einer Abfolge von Querstrom-Thormannböden die Flüssigkeit auf einem tiefer gelegenen Querstrom-Thormannboden nicht auf direktem Weg quer über den Boden, sondern auf vorstehend beschriebene Weise angetrieben mäandrierend von dem wenigstens einen Zulauf zu dem wenigstens einen Ablauf. Der Raum zwischen zwei in Querstromrichtung hintereinander angeordneten Hauben eines Querstrom-Thormannbodens bildet jeweils eine Rinne, in der die Flüssigkeit fließt. Die Detailausgestaltung eines Querstrom-Thormannbodens erfolgt darüber hinaus normalerweise so, dass die Flüssigkeit in zwei in Querstromrichtung jeweils aufeinanderfolgenden Rinnen im Gegenstrom fließt (vgl. z. B. Figur 3 der DE 10243625 A1). Das auf diese Weise resultierende Mäandrieren der Querströmung verlängert den Strömungsweg der Flüssigkeit von dem wenigstens einen Zulauf zu dem wenigstens einen Ablauf, was die Trennwirkung eines Querstrom-Thormannbodens begünstigt.

Wie bereits ausgeführt, wird bei einem Querstrom-Glockenboden das aus der Glocke austretende Gas, im Unterschied zum Querstrom-Siebboden, parallel zur Bodenfläche in die auf dem Querstrom-Glockenboden aufgestaute Flüssigkeit eingeleitet. Reibungs- und Auftriebskräfte sorgen dann dafür, dass mit zunehmendem Abstand des ausgetretenen Gasstroms vom Glockenrand immer mehr Teilströme desselben in eine Richtung senkrecht zum Querstrom-Glockenboden umgelenkt werden und schließlich aus der Flüssigkeitsschicht ausdringen. Mit zunehmender Gasbelastung einer Glocke wächst die Geschwindigkeit des aus ihr austretenden Gasstroms, was den Abstand vom Rand der Glocke ("den Wirkungsbereich der Glocke"), bis zu welchem vorstehend beschriebene Umlenkung erfolgt ist, vergrößert.

Dieser Abhängigkeit des Wirkungsbereichs einer starren Glocke von der Gasbelastung kann dadurch entgegengewirkt werden, dass die Durchtrittsöffnung eines Querstrom-Stoffaustauschbodens als Ventil (als Ventildurchtrittsöffnung) ausgestaltet (ausgeführt) wird. Die dabei resultierenden Querstrom-Stoffaustauschböden werden als Querstrom-Ventilböden bezeichnet (vgl. z. B. DD 279822 A1, DD 216633 A1 und DE 102010001228 A1).

Der Begriff Querstrom-Ventilböden subsumiert in dieser Schrift somit Querstrom-Stoffaustauschböden, die Durchtrittsöffnungen (Bodenbohrungen) mit hubbegrenzten Teller-, Ballast- oder Hebeventilen (Schwimmklappen) aufweisen, die die Größe der Dampfdurchtrittsöffnung der jeweiligen Kolonnenbelastung anpassen.

In einer einfachen Ausgestaltung werden die Durchtrittsöffnungen des Bodens zu vorgenanntem Zweck mit nach oben beweglichen Deckeln oder Tellern (Scheiben) abgedeckt. Beim Durchtritt des aufsteigenden Gases werden die Deckel (Teller, Scheiben) durch den Gasstrom in einem über die jeweilige Durchtrittsöffnung zusätzlich angebrachten (das normalerweise am Boden fest verankert ist) entsprechenden Führgerüst (Führkäfig) angehoben und erreichen schließlich eine der Gasbelastung entsprechende Hubhöhe (anstelle eines Führkäfigs kann die Scheibe auch über mit dem Boden verankerte aufwärtsbewegliche Ventilbeine verfügen, deren Aufwärtsbeweglichkeit nach oben begrenzt ist). Der durch die Durchtrittsöffnung aufsteigende Gasstrom wird an der Unterseite des angehobenen Deckels (Tellers, Scheibe) in ähnlicher Weise wie in der Glocke (bei einer Glockendurchtrittsöffnung) umgelenkt und tritt aus dem unter dem angehobenen Teller (Deckel, Scheibe) entstandenen Austrittsbereich aus und wie beim Glockenboden parallel zum Boden in die auf selbigem aufgestaute Flüssigkeit ein. Der Tellerhub steuert so die Größe des Gasaustrittsbereichs und passt sich selbständig der Kolonnenbelastung an, bis das obere Ende des Führkäfigs die maximal mögliche Hubhöhe begrenzt. Die Teller können dabei nach unten gerichtete Distanzhalter aufweisen, sodass bei niedriger Gasbelastung das Ventil nur so weit schließt, dass der durch die Distanzhalter geschaffene Raum noch eine intensive Vermischung der horizontalen Gasausströmung mit der querströmenden Flüssigkeit gestattet. Distanzhalter wirken auch einem Anhaften der Ventilscheibe am Boden entgegen. Durch geeignete Ausgestaltung der Ventilelemente eines Querstrom-Ventilbodens kann die Blasrichtung des Ventilelements eingestellt und so die Flüssigkeitszwangsführung auf dem Querstrom-Ventilboden zusätzlich beeinflusst werden (vgl. z. B. DD 216 633 A1). Das Prinzip von Querstrom-Ventilböden sowie für die Zwecke der vorliegenden Schrift verwendbare Ventilböden findet sich z. B. in Technische Fortschrittsberichte, Band 61, Grundlagen der Dimensionierung von Kolonnenböden, Seite 96 bis 138 ausgeführt. Neben den vorstehend beschriebenen beweglichen Ventilen kennt der Fachmann auch noch feststehende Ventile. Dabei handelt es sich normalerweise um scheibenförmige, oder trapezförmige, oder rechteckige Einheiten, die aus der Bodenplatte herausgestanzt werden und mit dieser über nach oben gerichtete feststehende Beine verbunden sind.

Insbesondere bei größeren Durchmessern einer Trennkolonne bildet sich auf Querstrom-Stoffaustauschböden von dem wenigstens einen Zulauf ausgehend bis zum Erreichen des Ablaufwehrs des wenigstens einen Ablaufs in natürlicher Weise ein zu beachtendes Flüssigkeitsgefälle aus (der Gradient der Stauhöhe der Flüssigkeit speist (bedingt) die Querströmung). Dies hat zur Folge, dass in Bereichen mit einer geringeren Flüssigkeitshöhe aufgrund der daraus resultierenden geringeren Widerstände der aufsteigende Dampf (das aufsteigende Gas) vergleichsweise leichter durch die Flüssigkeitsschicht hindurchtreten kann. Daraus kann schließlich eine ungleichmäßige Gasbelastung des Querstrom-Stoffaustauschbodens erwachsen (die Bereiche mit einer geringeren Flüssigkeitshöhe (einem geringeren Durchströmungswiderstand) werden bevorzugt durchströmt), die die Trennwirkung desselben beeinträchtigt. Durch die Anwendung von z. B. in ihrer Sitzhöhe einstellbaren Glocken (alternativ kann auch die Glockengröße verändert werden) bei Querstrom-Glockenböden bzw. durch Verwendung von z. B. Tellern (Deckeln) mit unterschiedlichem Gewicht bei Querstrom-Ventilböden kann diesbezüglich ausgleichend eingewirkt werden, so dass der Stoffaustauschboden über seinen Querschnitt im Wesentlichen gleichmäßig gast (dort wo die Flüssigkeitshöhe auf dem Querstrom-Stoffaustauschboden kleiner ist, wird die Sitzhöhe der Glocke anwendungstechnisch zweckmäßig entsprechend tiefer liegend bzw. das Gewicht des Hubtellers (Hubdeckels) entsprechend höher liegend gewählt; die Sitzhöhe der Glocke kann z.B. auch dadurch tiefer gelegt werden, dass die Länge des entsprechenden Kamins, an dessen Ende die Glocke gegebenenfalls verschraubt aufsitzt, gezielt verkürzt wird; alternativ oder zusätzlich kann z.B. auch die Zacken-/ Schlitzstruktur des Glockenrands variiert werden, um den erwünschten Strömungswiderstandsausgleich zu bewirken; idealer Weise erfolgt die Einstellung über den Querstrom-Stoffaustauschboden so, dass im Betrieb der Trennkolonne jede der auf einem Querstrom-Glockenboden befindliche Glocke den gleichen Strömungswiderstand für das aufsteigende Gas bedingt). Im Übrigen sind die Durchtrittstellen (die Durchtrittsöffnungen) eines Querstrom-Stoffaustauschbodens in der Regel vorteilhaft einheitlich gestaltet.

Durch einen Querstrom-Stoffaustauschboden hindurch verlaufende Öffnungen (von oben nach unten), deren Querschnittsfläche üblicherweise mehr als 200 mal kleiner als die Gesamtquerschnittsfläche aller übrigen Öffnungen des Querstromstoffaustauschbodens (den Querschnitt des wenigstens einen Ablaufschachtes nicht miteinbezogen) ist, bilden keine (trennwirksamen) Durchtrittsöffnungen für das durch den Querstrom-Stoffaustauschboden aufsteigende Gas und werden selbigen daher nicht zugerechnet. Beispielsweise kann es sich bei solchen Öffnungen um winzige Leerlaufbohrungen handeln, über die hydraulisch abgedichtete Querstromböden beim Abschalten einer Trennkolonne leerlaufen können. Auch können solche Öffnungen Verschraubungszwecken dienen.

Abfolgen von wenigstens einen Ablaufschacht aufweisenden Stoffaustauschböden, bei denen sich der wenigstens eine Zulauf und der wenigstens eine Ablauf z. B. in der gleichen Hälfte des (kreisförmigen) Stoffaustauschbodens befinden oder bei denen sich der wenigstens eine Zulauf in Bodenmitte und der wenigstens eine Ablauf am Rand des Bodens befindet, bilden keine Abfolge von Querstrom-Stoffaustauschböden im anmeldegemäßen (erfindungsgemäßen) Sinn.

Der Wirkungsgrad von wie beschrieben ausgeführten Querstrom-Stoffaustauschböden liegt üblicherweise unterhalb desjenigen eines theoretischen Bodens (einer theoretischen Trennstufe). Als theoretischer Boden (oder theoretische Trennstufe) soll in dieser Schrift ganz generell diejenige Raumeinheit einer für ein thermisches Trennverfahren eingesetzten, trennwirksame Einbauten enthaltenden, Trennkolonne verstanden werden, die eine Stoffanreicherung entsprechend dem thermodynamischen Gleichgewicht bewirkt. D. h., der Begriff des theoretischen Bodens ist sowohl auf Trennkolonnen mit Stoffaustauschböden, als auch auf Trennkolonnen mit Packungen und/oder Füllkörpern anwendbar.

Der Stand der Technik empfiehlt den Einsatz von Abfolgen von wenigstens zwei baugleichen (identisch ausgeführten) Querstrom- Stoffaustauschböden u. a. in trennwirksame Einbauten enthaltenden Trennkolonnen, die zur Durchführung thermischer Trennverfahren zwischen wenigstens einem in der Trennkolonne aufsteigenden Gasstrom und wenigstens einem in der Trennkolonne absteigenden Flüssigkeitsstrom zur Anwendung kommen, und wobei wenigstens einer der Ströme wenigstens ein (Meth)acrylmonomeres enthält. Beispielsweise empfehlen die Schriften DE 19924532 A1, DE 10243625 A1 und WO 2008/090190 A1 die Mitverwendung einer Abfolge baugleicher hydraulisch abgedichteter Querstrom-Stoffaustauschböden in einer Trennkolonne zur Durchführung eines Verfahrens der fraktionierenden Kondensation eines Acrylsäure enthaltenden Produktgasgemischs einer heterogen katalysierten Gasphasen-Partialoxidation von $C_3$-Vorläufern der Acrylsäure mit molekularem Sauerstoff, die von unten nach oben zunächst Dual-Flow-Böden und im Anschluss daran hydraulisch abgedichtete Querstrom-Stoffaustauschböden enthält.

Charakteristisch für die Abfolgen der im Stand der Technik empfohlenen Querstrom-Stoffaustauschböden ist, dass der jeweils untere von zwei in der Abfolge aufeinanderfolgenden Querstrom-Stoffaustauschböden in Richtung der Querströmung von seinem wenigstens einen Zulauf zu seinem wenigstens einen Ablaufschacht nur im Bereich zwischen dem wenigstens einen Zulauf und dem wenigstens einen Ablaufschacht (der wenigstens einen Ablauföffnung) Durchtrittsöffnungen aufweist (vgl. z. B. die Figuren 3 und 4 der DE 10243625 A1, die Figur 1 der DD 279822 A1, die Figur 1 der DD 216633 A1 und die Abbildung 1 aus Chemie-Ing.-Techn. 45. Jahrgang, 1973/Nr. 9 + 10, Seite 617 bis 620).

In der EP 2460577 A1 ist eine Ölwaschkolonne beschrieben. Bei dieser Ölwaschkolonne wird über einen seitlichen Abzug eine gesammelte Flüssigphase abgezogen und in einen Vorratsbehälter geführt. Aus dem Vorratsbehälter wird die über einen Wärmetauscher abgekühlte reine Flüssigphase dem Benzinabschnitt als Rücklauf wieder aufgegeben. Die Rückführung erfolgt dabei auf einem Ventilboden, welcher zwei Böden über dem untersten Ventilboden angeordnet ist, bei welchem der seitliche Abzug angeordnet ist.

Die CA 2017980 A1 beschreibt einen Destillationsreaktor, bei dem ein Katalysator durch einen frischen oder regenerierten Katalysator ersetzt wird. Hierfür wird der flüssige Katalysator, der sich auf einem Auffangboden gesammelt hat, über einen Auslass entnommen. Der entnommene Katalysator wird einem Separator zugeführt, bei dem feste Bestandteile des Katalysators abgesondert werden. Der flüssige Katalysator wird zurück in den Destillationsreaktor gepumpt. Hierfür ist eine Rückführöffnung vorgesehen, die unterhalb der Entnahme des Katalysators angeordnet ist.

Des Weiteren beschreibt die DE 19924532 A1 ein Verfahren der fraktionierten Kondensation eines Acrylsäure enthaltenden Produktgasgemisches einer heterogenen katalysierten Gasphasen-Partialoxidation von C3-Vorläufern der Acrylsäure mit molekularem Sauerstoff. Die verwendete Trennkolonne weist sowohl Dual-Flow-Böden als auch hydraulisch abgedichtete Querstromböden als trennwirksame Einbauten auf.

Eine weitere Kolonne zum Trennen von Staub ist in der US 2,141,829 beschrieben.

Schließlich beschreibt die EP 00009545 ein Verfahren zur Gewinnung von Acrylsäure aus einem gasförmigen Reaktorabstrom, der Acrylsäure, Acrolein, Wasser und Verunreinigungen enthält.

Eine problematische Eigenschaft von (Meth)acrylmonomeren ist deren Neigung zu unerwünschter Polymerisation, die sich, insbesondere in flüssiger Phase befindlich, auch durch den Zusatz von Polymerisationsinhibitoren nicht vollständig unterdrücken lässt.

Nachteilig an bekannten Trennkolonnen ist, dass es bei kontinuierlicher Durchführung des thermischen Trennverfahrens während längerer Betriebsdauern bei den Stoffaustauschböden vergleichsweise häufig zur Ausbildung von unerwünschtem Polymerisat kommt. Dies ist insbesondere deshalb von Nachteil, weil der Betreiber des thermischen Trennverfahrens aufgrund der unerwünschten Polymerisatbildung das thermische Trennverfahren immer wieder unterbrechen muss, um das gebildete Polymerisat zu entfernen. Selbiges kann nämlich die Durchtrittsöffnungen des Stoffaustauschbodens teilweise oder vollständig verschließen. Außerdem ist die radikalische Polymerisation von (Meth)acrylmonomere normalerweise ausgeprägt exotherm, d. h. unter starker Wärmeentwicklung. Es besteht die Gefahr, dass die Polymerisation so heftig verläuft, dass die Trennkolonne, welche das Polymerisationsgemisch enthält, explodiert.

Der vorliegenden Erfindung lag daher die Aufgabe zugrunde, eine Kolonne und ein thermisches Trennverfahren der eingangs genannten Art bereitzustellen, bei denen eine Polymerisation des in der Trennkolonne befindlichen Stoffs verhindert oder zumindest verringert werden kann.

Erfindungsgemäß wird diese Aufgabe durch eine Kolonne mit den Merkmalen des Anspruchs 1 sowie ein thermisches Trennverfahren mit den Merkmalen des Anspruchs 13 gelöst. Vorteilhafte Ausgestaltungen und Weiterbildungen ergeben sich aus den abhängigen Ansprüchen.

Demgemäß betrifft die Erfindung eine Kolonne zur thermischen Behandlung eines Fluids mit einem zylindrischen, vertikal ausgerichteten Kolonnenkörper, der einen Kolonnenhohlraum bildet, und einem in dem Kolonnenhohlraum angeordneten Stoffaustauschboden, der eine Auffangfläche bildet. Die erfindungsgemäße Kolonne umfasst ferner eine Zirkulationseinrichtung mit zumindest einer in dem Kolonnenkörper oberhalb der Auffangfläche ausgebildeten Ablauföffnung, einer mit der Ablauföffnung in Fluidverbindung stehenden Zirkulationsleitung und zumindest einer mit der Zirkulationsleitung in Fluidverbindung stehenden, in dem Kolonnenkörper oberhalb der Auffangfläche ausgebildeten Rückführöffnung.

Die räumlichen Begriffe "oben", "unten", "horizontal" und "vertikal", beziehen sich, soweit nichts anderes ausdrücklich erwähnt ist, auf die Orientierung der Kolonne während des Betriebs.

Es wurde gefunden, dass sich das unerwünschte Polymerisat insbesondere in sogenannten Totzonen des Stoffaustauschbodens bildet. Bei solchen Totzonen ist die Verweilzeit des Fluids bei dem Stoffaustauschboden besonders lang. Eine solche lange Verweilzeit begünstigt die Polymerisation. Durch die bei der erfindungsgemäßen Kolonne vorgesehene Zirkulationseinrichtung kann eine Flüssigkeitszirkulation auf der Auffangfläche des Stoffaustauschbodens erzeugt werden, welche die Bildung von Totzonen vermeidet. Der Flüssigkeitsstrom nimmt etwaige Fluidrückstände mit. Die Verweilzeit von Flüssigkeit auf der Auffangfläche des Stoffaustauschbodens wird hierdurch reduziert. Es wird insbesondere die Verweilzeitverteilung enger, das heißt es gibt weniger Flüssigkeitsvolumina, die für eine längere Zeit auf dem Stoffaustauschboden verweilen. Vorteilhafterweise kann hierdurch verhindert werden, dass das Polymerisat Durchtrittsöffnungen des Stoffaustauschbodens teilweise oder vollständig verschließt. Außerdem wird die von dem Polymerisationsgemisch verursachte Explosionsgefahr verringert.

Gemäß einer Ausgestaltung der erfindungsgemäßen Kolonne ist die Ablauföffnung unmittelbar oberhalb des tiefsten Bereichs der Auffangfläche des Stoffaustauschbodens angeordnet. Der untere Rand der Ablauföffnung kann insbesondere auf derselben Höhe angeordnet sein, wie der tiefste Bereich der Auffangfläche, so dass die auf der Auffangfläche befindliche Flüssigkeit im Wesentlichen vollständig zur Ablauföffnung laufen kann, ohne dass Rückstände auf der Auffangfläche verbleiben. Vorteilhafterweise wird hierdurch effektiv die Polymerisatbildung verhindert, wenn in der Kolonne ein Fluid behandelt wird, welches zur Polymerisation neigt.

Gemäß einer Ausgestaltung der erfindungsgemäßen Kolonne sind in dem Kolonnenhohlraum mehrere vertikal beabstandete Stoffaustauschböden angeordnet und die Ablauföffnung und die Rückführöffnung sind vertikal zwischen zwei benachbarten vertikal beabstandeten Stoffaustauschböden angeordnet. In diesem Fall liegt insbesondere das Verhältnis des vertikalen Abstands des tiefsten Bereichs der Auffangfläche des Stoffaustauschbodens von der unteren Kante der Ablauföffnung zu dem vertikalen Abstand des tiefsten Bereichs der Auffangfläche des Stoffaustauschbodens von der Unterseite des darüber angeordneten Stoffaustauschbodens in einem Bereich von 0 bis 0,3, insbesondere in einem Bereich von 0 bis 0,1.

Die Rückführöffnung kann bei der erfindungsgemäßen Kolonne entweder oberhalb der Ablauföffnung in den Kolonnenhohlraum münden oder die Rückführöffnung mündet auf derselben Höhe wie die Ablauföffnung in den Kolonnenhohlraum. Vorteilhafterweise wird die Anordnung der Rückführöffnung relativ zu der Ablauföffnung so gewählt, dass auf der Auffangfläche des Stoffaustauschbodens eine Flüssigkeitszirkulation erzeugt wird, welche alle Bereiche des Stoffaustauschbodens erreicht, so dass sich insbesondere keine Totzonen bilden.

Gemäß einer Ausgestaltung der erfindungsgemäßen Kolonne liegt das Verhältnis des vertikalen Abstands des tiefsten Bereichs der Auffangfläche des Stoffaustauschbodens von der oberen Kante der Rückführöffnung zu dem vertikalen Abstand des tiefsten Bereichs der Auffangfläche des Stoffaustauschbodens von der Unterseite des unmittelbar darüber angeordneten Stoffaustauschbodens in einem Bereich von 0 bis 0,3, insbesondere in einem Bereich von 0 bis 0,2. Durch diese geometrischen Verhältnisse wird vorteilhafterweise gewährleistet, dass die Flüssigkeit üblicherweise unterhalb des Flüssigkeitsspiegels auf der Auffangfläche eingeleitet wird. Auch die Rückführöffnung ist insbesondere unmittelbar oberhalb des tiefsten Bereichs der Auffangfläche des Stoffaustauschbodens angeordnet. Hierdurch kann vorteilhafterweise eine besonders gute Durchmischung der Flüssigkeit auf der Auffangfläche des Stoffaustauschbodens erzielt werden.

Gemäß einer Weiterbildung der erfindungsgemäßen Kolonne ist in der Rückführöffnung eine Düse zum Erzeugen eines Flüssigkeitsstrahls beim Eintritt der Flüssigkeit in den Kolonnenhohlraum angeordnet. Sind bei der Kolonne mehrere Rückführöffnungen angeordnet, kann insbesondere jede dieser Rückführöffnungen eine solche Düse aufweisen. Mittels der Düse oder der Düsen kann noch wirkungsvoller verhindert werden, dass Totzonen entstehen, bei denen sich Polymerisat bei der Behandlung eines entsprechenden Fluids bildet.

Die Zirkulationsleitung und die Rückführöffnung bzw. die Rückführöffnungen sind insbesondere so angeordnet, dass die rückgeführte Flüssigkeit radial in den Kolonnenhohlraum eintritt. Vorteilhafterweise wird hierdurch noch wirkungsvoller die Bildung von Totzonen vermieden.

Gemäß einer Weiterbildung der erfindungsgemäßen Kolonne sind mehrere voneinander beabstandete Rückführöffnungen in dem Kolonnenkörper ausgebildet. Diese Rückführöffnungen stehen jeweils in Fluidverbindung mit der Zirkulationsleitung. Die über die Ablauföffnung abgeführte Flüssigkeit kann auf diese Weise bei mehreren Stellen dem Kolonnenhohlraum oberhalb des Stoffaustauschbodens wieder zugeführt werden. Vorteilhafterweise wird hierdurch noch effektiver erreicht, dass sich keine Totzonen bei dem Auffangboden bilden.

Bei der erfindungsgemäßen Kolonne weist der Stoffaustauschboden bzw. zumindest ein Boden der Kolonne Durchtrittsöffnungen für von unten aufsteigendes Gas auf. Bei diesen Durchtrittsöffnungen erstrecken sich zylindrische Körper nach oben. Diese zylindrischen Körper sind insbesondere in einem gewissen Abstand mit Hauben, Kappen oder Dächern versehen, so dass Gas aus den zylindrischen Körpern seitlich austreten kann, ohne dass die vom darüber liegenden Stoffaustauschboden abregnende Flüssigkeit sich im Gegenstrom zum Gas bewegen kann. Diese auch als Kamine bezeichneten, auf dem Stoffaustauschboden senkrecht stehenden Körper können in Sonderfällen neben einem kreisförmigen Querschnitt auch eine quadratische, rechteckige, elliptische Querschnittsfläche haben. Der Abstand (A) der Hauben, Kappen oder Dächern zum zylindrischen Körper mit Durchmesser (D) beträgt dabei in der Regel A < 2D. Bei dieser Art von Boden handelt es sich um einen Kaminboden, der zu mindestens einen Teil der vom oberen Boden abregenden Flüssigkeit im Raum zwischen den Kaminen sammelt, wo sie von dort einer gezielten anderen Verwendung zugeführt werden kann.

Gemäß einer Ausgestaltung der erfindungsgemäßen Kolonne bilden die oberen Kanten des zylindrischen Körpers Überlaufkanten. Das Verhältnis des vertikalen Abstands des tiefsten Bereichs der Auffangfläche des Stoffaustauschbodens von der unteren Kante der Ablauföffnung zu dem vertikalen Abstand des tiefsten Bereichs der Auffangfläche des Stoffaustauschbodens von der Höhe der untersten Überlaufkante der zylindrischen Körper liegt dabei z. B. in einem Bereich von 0 bis 0,1, insbesondere in einem Bereich von 0 bis 0,05. Bei der erfindungsgemäßen Kolonne liegt das Verhältnis des vertikalen Abstands des tiefsten Bereichs der Auffangfläche des Stoffaustauschbodens von der oberen Kante der Rückführöffnung zu dem vertikalen Abstand des tiefsten Bereichs der Auffangfläche des Stoffaustauschbodens von der Höhe der untersten Überlaufkante der zylindrischen Körper in einem Bereich von 0 bis 0,9, insbesondere in einem Bereich von 0 bis 0,3. Durch diese geometrischen Verhältnisse wird vorteilhafterweise gewährleistet, dass die Flüssigkeit üblicherweise unterhalb des Flüssigkeitsspiegels auf der Auffangfläche eingeleitet wird. Hierdurch kann vorteilhafterweise eine besonders gute Durchmischung der Flüssigkeit in den Bereichen zwischen den zylindrischen Körpern erzielt werden.

Bei einer erfindungsgemäßen Ausgestaltung dieses Bodens sind die Rückführöffnungen so relativ zu den zylindrischen Körpern angeordnet, dass sich ein mäanderförmiger Flüssigkeitsstrom auf der Auffangfläche des Stoffaustauschbodens ergibt. Der Flüssigkeitsstrom kann auf diese Weise insbesondere alle Bereiche der Auffangfläche auf den Stoffaustauschboden erreichen, so dass sich trotz der Unterbrechungen durch die zylindrischen Körper keine Totzonen auf der Auffangfläche bilden.

Gemäß einer Weiterbildung der erfindungsgemäßen Kolonne weist die Zirkulationsleitung eine Einmündung zum Zuführen einer weiteren Flüssigkeit auf. Über diese Einmündung wird insbesondere eine inerte Flüssigkeit oder eine Flüssigkeit mit Polymerisationsinhibitoren zugeführt.

In der Zirkulationsleitung ist insbesondere eine Pumpe angeordnet, mit welcher sich auf der Auffangfläche des Stoffaustauschbodens ansammelnde Flüssigkeit abpumpbar und diese durch die Rückführöffnung dem Kolonnenhohlraum wieder zuführbar ist. Mittels der Pumpe oder eines zusätzlichen Regelventils kann der Flüssigkeitsstrom auf dem Stoffaustauschboden gesteuert oder geregelt werden. Die Pumpe oder das Regelventil kann ferner mit einem Sensor gekoppelt sein, welcher den Flüssigkeitsstand auf der Auffangfläche des Stoffaustauschbodens misst. In Abhängigkeit von diesem Flüssigkeitsstand kann die Pumpe dann die Zirkulation und/oder die Flüssigkeitsabfuhr der Flüssigkeit regeln.

Die erfindungsgemäße Kolonne kann insbesondere als Trennkolonne eingesetzt werden. Die Trennkolonne weist eine Abfolge von Stoffaustauschböden auf. Als Stoffaustauschböden werden insbesondere die eingangs genannten Böden verwendet, d. h. Stoffaustauschböden ohne Zwangsführung, wie Regensiebböden und Dual-Flow-Böden, und Stoffaustauschböden mit einer Flüssigkeitszwangsführung, wie z. B. Querstrom-Stoffaustauschböden, insbesondere Querstrom-Glockenböden, Querstrom-Haubenböden, Querstrom-Thormannböden und Querstrom-Ventilböden.

Der lichte Abstand zwischen zwei innerhalb der erfindungsgemäßen Kolonne unmittelbar aufeinanderfolgenden Böden beträgt insbesondere nicht mehr als 700 mm, vorzugsweise nicht mehr als 600 mm bzw. nicht mehr als 500 mm. Anwendungstechnisch zweckmäßig ist der lichte Abstand innerhalb der Bodenabfolge 300 bis 500 mm. Im Regelfall sollte der Bodenabstand 250 mm nicht unterschreiten.

Die Höhe des Kolonnenkörpers ist beispielsweise größer als 5 m, insbesondere größer als 10 m. Es ist jedoch auch möglich, dass die Höhe des Kolonnenkörpers 30 m oder 40 m übersteigt.

Zwischen den Böden können weitere trennwirksame Einbauten angeordnet sein. Durch die trennwirksamen Einbauten wird die Stofftrennung in der Trennkolonne verbessert. Diese weiteren Einbauten können beispielsweise in Form von Packungen, insbesondere strukturierten bzw. geordneten Packungen, und/oder Schüttungen vorgesehen sein. Unter den Schüttungen sind solche mit Ringen, Wendeln, Sattelkörpern, Raschig-, Intos- oder Pall-Ringen, Berl- oder Intalox-Sätteln, Top-Pak etc. bevorzugt. Für erfindungsgemäß zu verwendende Extraktionskolonnen besonders geeignete Packungen sind z. B. Packungen der Julius Montz GmbH in D-40705 Hilden, wie z. B. die Packung Montz-Pak B1-350. Vorzugsweise verwendet man gelochte strukturierte Packungen aus Edelstahlblechen. Packungskolonnen mit geordneten Packungen sind dem Fachmann an sich bekannt und z. B. in Chem.-Ing.Tech. 58 (1986) Nr. 1, S. 19 - 31 sowie in der Technischen Rundschau Sulzer 2/1979, S. 49 ff. der Gebrüder Sulzer Aktiengesellschaft in CH-Winterthur beschrieben.

Die Erfindung betrifft des Weiteren ein thermisches Trennverfahren zwischen wenigstens einem in einer Kolonne, wie sie vorstehend beschrieben wurde, aufsteigenden Gas und wenigstens einer in der Kolonne absteigenden Flüssigkeit.

Gemäß einer Ausgestaltung des erfindungsgemäßen Verfahrens wird die über die Rückführöffnung eingeleitete Flüssigkeit unterhalb des Flüssigkeitsstandes auf der Auffangfläche eingeleitet.

Gemäß einer Ausgestaltung des erfindungsgemäßen Verfahrens enthält das aufsteigende Gas und/oder die absteigenden Flüssigkeit insbesondere (Meth)acrylmonomere.

Das erfindungsgemäße thermische Trennverfahren kann z.B. ein Verfahren der fraktionierenden Kondensation zur Abtrennung von Acrylsäure aus einem Acrylsäure enthaltenden Produktgasgemisch einer heterogen katalysierten Gasphasenpartialoxidation einer C3-Vorläuferverbindung (insbesondere Propen und/oder Propan) der Acrylsäure mit molekularem Sauerstoff zu Acrylsäure sein.

Die Trennkolonne (Kondensationskolonne) kann wie in den Schriften DE 10243625 A1 bzw. WO 2008/090190 A1 beschrieben ausgeführt sein, sieht man davon ab, dass bei den dort eingesetzten Böden zumindest zum Teil die vorstehend beschriebenen Zirkulationseinrichtungen vorgesehen sind.

Bei dem erfindungsgemäßen Verfahren ist aufgrund der Verwendung von (Meth)acrylmonomeren die Neigung zur Polymerisation besonders groß. Eine solche unerwünschte Polymerisation wird bei dem erfindungsgemäßen Verfahren dadurch verhindert, dass die sich auf der Auffangfläche eines Stoffaustauschbodens ansammelnde Flüssigkeit durch die Ablauföffnung abgepumpt wird und über die Rückführöffnung oder über die Rückführöffnungen dem Kolonnenhohlraum wieder zugeführt wird.

Im Folgenden werden Ausführungsbeispiele der erfindungsgemäßen Kolonne und Ausführungsbeispiele des erfindungsgemäßen Verfahrens mit Bezug zu den Zeichnungen erläutert.

Figur 1     zeigt schematisch einen Vertikalschnitt eines Teils einer Kolonne gemäß einem Ausführungsbeispiel der Erfindung,

Figur 2     zeigt schematisch einen Vertikalschnitt eines Teils einer Kolonne gemäß einem weiteren Ausführungsbeispiel der Erfindung,

Figur 3     veranschaulicht die vertikale Anordnung von Einrichtungen bei der Kolonne des weiteren Ausführungsbeispiels und

Figur 4     zeigt einen Querschnitt der Kolonne eines noch weiteren Ausführungsbeispiels der Erfindung.

Das im Folgenden beschriebene Ausführungsbeispiel betrifft eine Trennkolonne 1, wie sie z. B. bei einem Verfahren der fraktionierenden Kondensation zur Abtrennung von Acrylsäure aus einem Acrylsäure enthaltenden Produktgasgemisch einer heterogen katalysierten Gasphasenpartialoxidation einer C3-Vorläuferverbindung (insbesondere Propen und/oder Propan) der Acrylsäure mit molekularem Sauerstoff zu Acrylsäure eingesetzt wird.

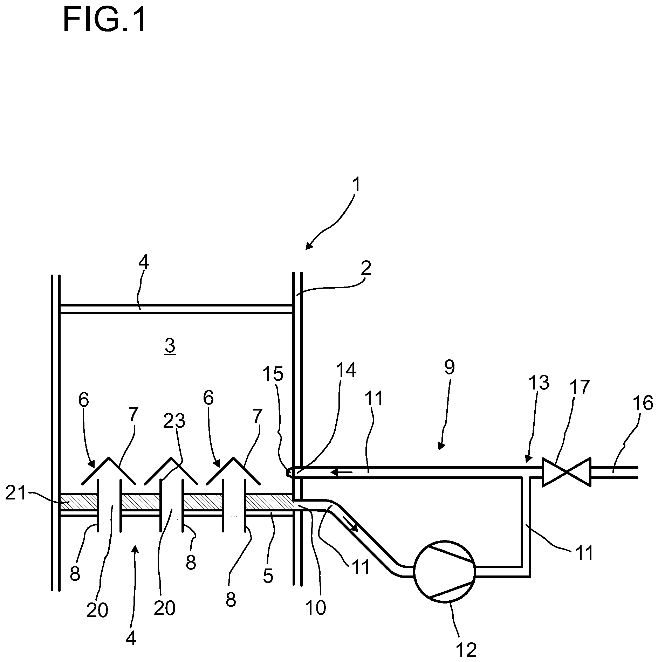

In Fig. 1 ist die an sich bekannte Trennkolonne 1 schematisch dargestellt. Sie umfasst einen zylindrischen Kolonnenkörper 2, dessen Achse vertikal ausgerichtet ist. Bei dem Kolonnenkörper 2 handelt es sich im Wesentlichen um einen Hohlzylinder. Das heißt, der Mantel des Kolonnenkörpers 2 bildet einen Kolonnenhohlraum 3. Der Kolonnenkörper 2 ist aus Edelstahl gefertigt. Nach außen ist die Trennkolonne 1 normalerweise in herkömmlicher Weise thermisch isoliert. Die Höhe der Trennkolonne 1 ist 40 m.

Im Kolonnenhohlraum 3 sind mehrere Stoffaustauschböden 4 befestigt, die horizontal ausgerichtet und vertikal voneinander beabstandet sind. Die Stoffaustauschböden 4 dienen als trennwirksame Einbauten, welche die Stofftrennung in der Trennkolonne 1 verbessern. Bei der in Figur 1 gezeigten Teilansicht ist einer der Stoffaustauschböden 4 gezeigt.

Bei dem Stoffaustauschboden 4 handelt es sich in diesem Fall um einen Kaminboden. Dieser Stoffaustauschboden 4 umfasst eine horizontal in dem Kolonnenkörper 2 befestigte Platte, welche auf der Oberseite die Auffangfläche 5 bildet. In der Platte sind Durchtrittsöffnungen 20 gebildet. In diese Durchtrittsöffnungen 20 sind Kamine 6 eingesetzt. Dabei sind in die Durchtrittsöffnungen 20 fluiddicht zylindrische Körper 8, die auch als Kaminkörper bezeichnet werden, eingesetzt. Bis zur oberen Kante 23 der zylindrischen Körper 8 kann sich auf der Auffangfläche 5 Flüssigkeit 21 ansammeln. Die innerhalb des zylindrischen Körpers 8 gebildete Öffnung wird jeweils von einer Abdeckhaube 7 abgedeckt, das heißt die Öffnung wird gegen herabtropfende Flüssigkeit abgeschirmt. Derartige Kaminböden sind an sich bekannt.

Bei der erfindungsgemäßen Kolonne 1 ist nun eine Zirkulationseinrichtung 9 vorgesehen. Diese Zirkulationseinrichtung 9 umfasst eine Ablauföffnung 10, welche unmittelbar oberhalb der Auffangfläche 5 im Mantel des Kolonnenkörpers 2 gebildet ist. An die Ablauföffnung 10 schließt sich eine Zirkulationsleitung 11 an. Durch die Ablauföffnung 10 wird Flüssigkeit 21, die sich auf der Auffangfläche 5 ansammelt, aus den Kolonnenhohlraum 3 herausgeführt.

In der Zirkulationsleitung 11 ist eine Pumpe 12 angeordnet, welche die herausgeführte Flüssigkeit über die Zirkulationsleitung 11 zu einer Rückführöffnung 14 fördert. Die Rückführöffnung 14 ist oberhalb der oberen Kante der zylindrischen Körper 8 der Kamine 6 angeordnet. Die Zirkulationsleitung 11 mündet in diese Rückführöffnung 14, wobei in der Rückführöffnung 14 bzw. am Ende der Zirkulationsleitung 11 eine Düse 15 angeordnet ist. Über die Düse 15 wird die Flüssigkeit oberhalb der Ablauföffnung 10 zurück auf den Stoffaustauschboden 4 eingedüst. Die Zirkulationsleitung 11 und die Rückführöffnung 14 sind so angeordnet, dass die rückgeführte Flüssigkeit radial in den Kolonnenhohlraum 3 eintritt. Die Düse 15 ist dabei so ausgebildet, dass bei allen Bereichen auf der Auffangfläche 5 des Stoffaustauschbodens 4 eine Flüssigkeitsströmung erzeugt wird, die verhindert, dass sich lange Verweilzeiten von Flüssigkeitsvolumina auf der Auffangfläche ergeben.

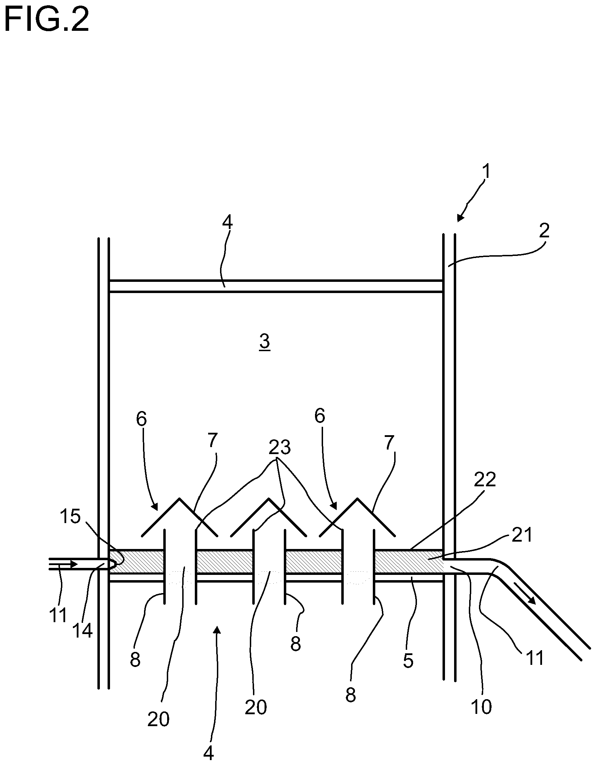

Gemäß einem weiteren in Figur 2 gezeigten Ausführungsbeispiel ist die Rückführöffnung 14 auf derselben Höhe wie die Ablauföffnung 10 angeordnet oder zumindest unterhalb der oberen Kante 23 der zylindrischen Körper 8 der Kamine 6. Die Rückführöffnung 14 ist in diesem Fall gegebenenfalls unterhalb des Flüssigkeitsspiegels 22 auf der Auffangfläche 5 angeordnet. In diesem Fall ist in der Rückführöffnung 14 eine sogenannte Treibstrahldüse 15 angeordnet, über welche die Flüssigkeit in die stehende Flüssigkeit 21 eingespritzt wird, wodurch eine Flüssigkeitsströmung auf der Auffangfläche 5 erzeugt wird.

Die weiteren Einrichtungen der Zirkulationseinrichtung 9 sind in Figur 2 aus Übersichtsgründen nicht dargestellt. Sie sind dieselben, wie sie bereits mit Bezug zu Figur 1 beschrieben wurden.

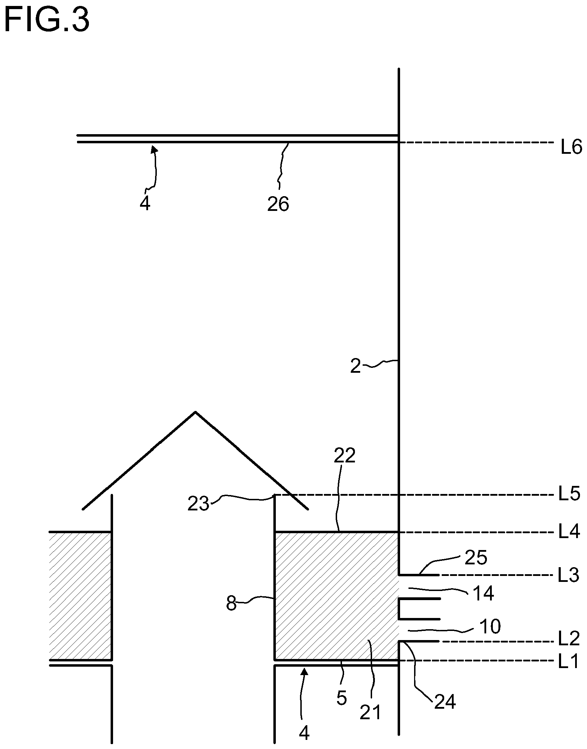

Anhand von Figur 3 werden mögliche vertikale Anordnungen der Auffangfläche 5, der Ablauföffnung 10, der Rückführöffnung 14, des Flüssigkeitsspiegels 22, der oberen Kanten 23 der zylindrischen Körper 8 und der Unterseite 26 des unmittelbar darüber angeordneten Stoffaustauschbodens 4 veranschaulicht. Die Auffangfläche 5 ist bei der Höhe L1, die untere Kante 24 der Ablauföffnung 10 ist bei der Höhe L2 und die obere Kante 25 der Rücköffnung 14 ist bei der Höhe L3 angeordnet. Der Flüssigkeitsspiegel 22 ist bei der Höhe L4 angeordnet. Die zylindrischen Körper 8 der Kamine 6 bilden bei ihren oberen Kanten 23 Überlaufkanten. Die unterste Überlaufkante der zylindrischen Körper 8 ist bei der Höhe L5 angeordnet. Die Unterseite 26 des unmittelbar über der Auffangfläche 5 angeordneten Stoffaustauschbodens 4 ist bei der Höhe L6 angeordnet.

Bei dem Ausführungsbeispiel der Figur 3 sind die Rückführöffnung 14 und die Ablauföffnung 10 zwischen den beiden benachbarten Stoffaustauschböden 4 und unterhalb des Flüssigkeitsspiegels 22 angeordnet. Des Weiteren liegt das Verhältnis des vertikalen Abstands der Auffangfläche 5 des Stoffaustauschbodens 4 von der unteren Kante 24 der Ablauföffnung 10 zu dem vertikalen Abstand der Auffangfläche 5 des Stoffaustauschbodens 4 von der Höhe L5 der untersten Überlaufkante der zylindrischen Körper 8 in einem Bereich von 0 bis 0,1, d.h. es gilt:

$$0 \leq \frac{abs(L1 - L2)}{abs(L1 - L5)} \leq 0,1$$

Ferner liegt das Verhältnis des vertikalen Abstands der Auffangfläche 5 des Stoffaustauschbodens 4 von der oberen Kante 25 der Rückführöffnung 14 zu dem vertikalen Abstand der Auffangfläche 5 des Stoffaustauschbodens 4 von der Höhe L5 der untersten Überlaufkante der zylindrischen Körper 8 in einem Bereich von 0 bis 0,9, d.h. es gilt:

$$0 \leq \frac{abs(L1 - L3)}{abs(L1 - L5)} \leq 0,9$$

Wenn der Auffangboden 4 kein Kaminboden sondern ein anders ausgebildeter Auffangboden 4 ist, der keinen zylindrischen Körper 8 aufweist, liegt das Verhältnis des vertikalen Abstands der Auffangfläche 5 des Stoffaustauschbodens 4 von der unteren Kante 24 der Ablauföffnung 10 zu dem vertikalen Abstand der Auffangfläche 5 des Stoffaustauschbodens 4 von der Unterseite 26 des unmittelbar darüber angeordneten Stoffaustauschbodens 4 in einem Bereich von 0 bis 0,3, d.h. es gilt:

$$0 \leq \frac{abs(L1 - L2)}{abs(L1 - L5)} \leq 0,3$$

Ferner liegt das Verhältnis des vertikalen Abstands der Auffangfläche 5 des Stoffaustauschbodens 4 von der oberen Kante 25 der Rückführöffnung 14 zu dem vertikalen Abstand der Auffangfläche 5 des Stoffaustauschbodens 4 von der Unterseite 26 des unmittelbar darüber angeordneten Stoffaustauschbodens 4 in einem Bereich von 0 bis 0,3, d.h. es gilt:

$$0 \leq \frac{abs(L1 - L3)}{abs(L1 - L5)} \leq 0,3$$

Gemäß weiteren Ausführungsbeispielen können auch andere Ausgestaltungen der Auffangfläche des Stoffaustauschbodens 4 relativ zu der Ablauföffnung 10 gewählt werden. Beispielsweise kann eine in den Stoffaustauschboden 4 gebildete Rinne in die Ablauföffnung 10 münden. Ferner kann die Zirkulationsleitung 11 in mehrere Rückführöffnungen 14 münden.

Die Zirkulationsleitung 11 weist des Weiteren eine Einmündung 13 auf. Bei dieser Einmündung 13 mündet eine Zuführleitung 16 für eine weitere Flüssigkeit in die Zirkulationsleitung 11 ein. Die Zuführleitung 16 besitzt ein Ventil 17, über welches die Flüssigkeitszufuhr in die Zirkulationsleitung 11 gesteuert oder geregelt werden kann.

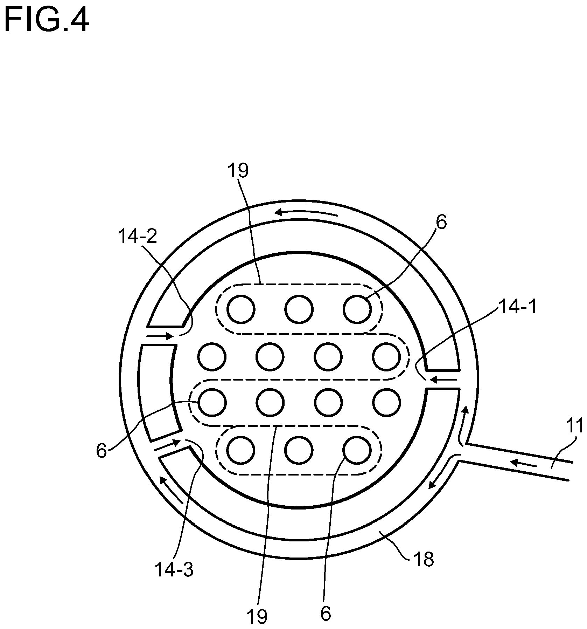

In Figur 4 ist ein Querschnitt auf der Höhe der Rückführöffnung 14 der erfindungsgemäßen Kolonne 1 gezeigt. Dabei zeigt Figur 4 ein noch weiteres Ausführungsbeispiel, bei dem nicht nur eine Rückführöffnung 14 im Kolonnenkörper 2 gebildet ist, sondern mehrere Rückführöffnungen 14-1, 14-2 und 14-3. Diese Rückführöffnungen 14-1 bis 14-3 sind über eine Ringleitung 18 mit der Zirkulationsleitung 11 verbunden. Die Rückführöffnungen 14-1 bis 14-3 sind in diesem Fall relativ zu den Kaminen 6 so angeordnet, dass sich eine mäanderförmige Flüssigkeitsströmung 19 auf der Auffangfläche 5 des Stoffaustauschbodens 4 ergibt. Die Rückführöffnungen 14-1, 14-2 und 14-3 sowie die Ablauföffnung 10 können dabei wie vorstehend mit Bezug zu Figur 3 erläutert in vertikaler Hinsicht angeordnet sein.

Es wird darauf hingewiesen, dass bei allen Ausführungsbeispielen auch andere der einleitend genannten Stoffaustauschböden verwendet werden können.

Im Folgenden wird ein Ausführungsbeispiel des erfindungsgemäßen Verfahrens beschrieben, welches mit der vorstehend beschriebenen Trennkolonne 1 eines der Ausführungsbeispiele ausgeführt wird.

Bei dem Verfahren handelt es sich um ein thermisches Trennverfahren zwischen wenigstens einem in der Trennkolonne 1 aufsteigenden Gas und wenigstens einer in der Trennkolonne 1 absteigenden Flüssigkeit. Dabei enthält das aufsteigende Gas und/oder die absteigenden Flüssigkeit insbesondere (Meth)acrylmonomere.

Bei dem Trennverfahren wird eine fraktionierende Kondensation zur Abtrennung von Acrylsäure aus einem Acrylsäure enthaltenden Produktgasgemisch einer heterogen katalysierten Gasphasenpartialoxidation einer C3-Vorläuferverbindung (insbesondere Propen und/oder Propan) der Acrylsäure mit molekularem Sauerstoff zu Acrylsäure in einer trennwirksame Einbauten enthaltenden Trennkolonne 1 durchgeführt. Die Trennkolonne 1 enthält von unten nach oben mehrere Stoffaustauschböden 4. Beispielsweise sind zunächst Dual-Flow-Böden und im Anschluss daran Querstrom-Haubenböden oder Kaminböden angeordnet, bei welchen zumindest zum Teil jeweils eine Zirkulationseinrichtung 9 ausgebildet ist, wie sie vorstehend beschrieben wurde. Ansonsten wird das Verfahren durchgeführt, wie es in den Schriften DE 19924532 A1, DE 10243625 A1 und WO 2008/090190 A1 beschrieben ist. Dabei wird jedoch kontinuierlich Flüssigkeit von den Auffangflächen 5 der Stoffaustauschböden 4 mittels der Zirkulationseinrichtungen 9 abgepumpt und der Auffangflächen 5 bei dem jeweiligen Stoffaustauschboden 4 über die Rückführöffnung 14 oder die Rückführöffnungen 14-1 bis 14-3 wieder zugeführt.

Unter dem Begriff "C3-Vorläufer" von Acrylsäure werden dabei solche chemischen Verbindungen zusammengefasst, die formal durch Reduktion von Acrylsäure erhältlich sind. Bekannte C3-Vorläufer von Acrylsäure sind z.B. Propan, Propen und Acrolein. Aber auch Verbindungen wie Glyzerin, Propionaldehyd, Propionsäure oder 3-Hydroxypropionsäure sind zu diesen C3-Vorläufern zu zählen. Von ihnen ausgehend handelt es sich bei der heterogen katalysierten Gasphasen-Partialoxidation mit molekularem Sauerstoff wenigstens teilweise um eine oxidative Dehydrierung. Bei den relevanten heterogen katalysierten Gasphasen-Partialoxidationen werden die genannten C3-Vorläufer der Acrylsäure, in der Regel mit inerten Gasen wie z.B. molekularer Stickstoff, CO, CO2, inerte Kohlenwasserstoffe und/oder Wasserdampf verdünnt, im Gemisch mit molekularem Sauerstoff bei erhöhten Temperaturen sowie gegebenenfalls erhöhtem Druck über übergangsmetallische Mischoxidkatalysatoren geleitet und oxidativ in ein Acrylsäure enthaltendes Produktgasgemisch umgewandelt.

In typischer Weise weist das Acrylsäure enthaltende Produktgasgemisch einer heterogen katalysierten Gasphasen-Partialoxidation von C3-Vorläufern (z.B. Propen) der Acrylsäure mit molekularem Sauerstoff an im festen Aggregatzustand befindlichen Katalysatoren, bezogen auf die Gesamtmenge der (in ihm) enthaltenen angegebenen Bestandteile, nachfolgende Gehalte auf:

| | |
|---|---|
| 1 bis 30 Gew.-% | Acrylsäure, |
| 0,05 bis 10 Gew.-% | molekularer Sauerstoff, |
| 1 bis 30 Gew.-% | Wasser, |
| 0 bis 5 Gew.-% | Essigsäure, |
| 0 bis 3 Gew.-% | Propionsäure, |
| 0 bis 1 Gew.-% | Maleinsäure und/oder Maleinsäure-Anhydrid, |
| 0 bis 2 Gew.-% | Acrolein, |
| 0 bis 1 Gew.-% | Formaldehyd, |
| 0 bis 1 Gew.-% | Furfural, |
| 0 bis 0,5 Gew.-% | Benzaldehyd, |
| 0 bis 1 Gew.-% | Propen, und |

als Restmenge inerte Gase wie z.B. Stickstoff, Kohlenmonoxid, Kohlendioxid, Methan und/oder Propan.

Die partielle Gasphasenoxidation selbst kann wie im Stand der Technik beschrieben durchgeführt werden. Ausgehend von Propen kann die partielle Gasphasenoxidation z.B. in zwei aufeinanderfolgenden Oxidationsstufen durchgeführt werden, wie sie z.B. in der EP 700 714 A1 und in der EP 700 893 A1 beschrieben sind. Selbstverständlich können aber auch die in der DE 19740253 A1 sowie in der DE 19740252 A1 zitierten Gasphasen-Partialoxidationen zur Anwendung kommen.

In der Regel beträgt die Temperatur des die partielle Gasphasenoxidation verlassenden Produktgasgemischs 150 bis 350°C, häufig 200 bis 300°C.

Durch direkte und/oder indirekte Kühlung wird das heiße Produktgasgemisch zweckmäßigerweise zunächst auf eine Temperatur von 100 bis 180°C abgekühlt, bevor es zum Zweck der fraktionierenden Kondensation in den Bereich C (den Sumpf) der Trennkolonne 1 geführt wird. Der in der Trennkolonne 1 herrschende Betriebsdruck beträgt in der Regel 0,5 bis 5 bar, häufig 0,5 bis 3 bar und vielfach 1 bis 2 bar.

Bezugszeichenliste:

| | |
|---|---|
| 1 | Kolonne, Trennkolonne |
| 2 | Kolonnenkörper |
| 3 | Kolonnenhohlraum |
| 4 | Stoffaustauschboden |
| 5 | Auffangfläche |
| 6 | Kamin |
| 7 | Abdeckhaube |
| 8 | zylindrischer Körper; Kaminkörper |
| 9 | Zirkulationseinrichtung |
| 10 | Ablauföffnung |
| 11 | Zirkulationsleitung |

| 12 | Pumpe |
|---|---|
| 13 | Einmündung |
| 14 | Rückführöffnung; |
| 14-1 bis 14-3 | Rückführöffnung |
| 15 | Düse; Treibstrahldüse |
| 16 | Zuführleitung |
| 17 | Ventil |
| 18 | Ringleitung |
| 19 | Flüssigkeitsströmung |
| 20 | Durchtrittsöffnungen |
| 21 | Flüssigkeit |
| 22 | Flüssigkeitsspiegel |
| 23 | obere Kante des zylindrischen Körpers |
| 24 | untere Kante der Ablauföffnung |
| 25 | obere Kante der Rückführöffnung |
| 26 | Unterseite des darüber angeordneten Stoffaustauschbodens |

**Patentansprüche**

1.  Kolonne (1) zur thermischen Behandlung eines Fluids mit einem zylindrischen, vertikal ausgerichteten Kolonnenkörper (2), der einen Kolonnenhohlraum (3) bildet, und einem in dem Kolonnenhohlraum (3) angeordneten Stoffaustauschboden (4), der eine Auffangfläche (5) bildet, und
    einer Zirkulationseinrichtung (9) mit zumindest einer in dem Kolonnenkörper (2) oberhalb der Auffangfläche (5) ausgebildeten Ablauföffnung (10), einer mit der Ablauföffnung (10) in Fluidverbindung stehenden Zirkulationsleitung (11) und zumindest einer mit der Zirkulationsleitung (11) in Fluidverbindung stehenden, in dem Kolonnenkörper (2) oberhalb der Auffangfläche (5) ausgebildeten Rückführöffnung (14; 14-1 bis 14-3),
    **dadurch gekennzeichnet, dass**
    der Stoffaustauschboden (4) Durchtrittsöffnungen (20) für von unten aufsteigendes Gas aufweist und sich bei den Durchtrittsöffnungen (20) zylindrische Körper (8) nach oben erstrecken, wobei
    die oberen Kanten (23) des zylindrischen Körper (8) Überlaufkanten bilden und das Verhältnis des vertikalen Abstands des tiefsten Bereichs (L1) der Auffangfläche (5) des Stoffaustauschbodens (4) von der oberen Kante (25) der Rückführöffnung (14; 14-1 bis 14-3) zu dem vertikalen Abstand des tiefsten Bereichs (L1) der Auffangfläche (5) des Stoffaustauschbodens (4) von der Höhe (L5) der untersten Überlaufkante der zylindrischen Körper (8) in einem Bereich von 0 bis 0,9 liegt, insbesondere in einem Bereich von 0 bis 0,3 liegt.

2.  Kolonne (1) nach Anspruch 1,
    **dadurch gekennzeichnet, dass**
    die untere Kante (24) der Ablauföffnung (10) unmittelbar oberhalb des tiefsten Bereichs der Auffangfläche (5) des Stoffaustauschbodens (4) angeordnet ist.

3.  Kolonne (1) nach Anspruch 1 oder 2,
    **dadurch gekennzeichnet, dass**
    in dem Kolonnenhohlraum (3) mehrere vertikal beabstandete Stoffaustauschböden (4) angeordnet sind und die Ablauföffnung (10) und die Rückführöffnung (14; 14-1 bis 14-3) vertikal zwischen zwei benachbarten vertikal beabstandeten Stoffaustauschböden (4) angeordnet sind.

4.  Kolonne (1) nach Anspruch 3,
    **dadurch gekennzeichnet, dass**
    das Verhältnis des vertikalen Abstands des tiefsten Bereichs (L1) der Auffangfläche (5) des Stoffaustauschbodens (4) von der unteren Kante (24) der Ablauföffnung (10) zu dem vertikalen Abstand des tiefsten Bereichs (L1) der Auffangfläche (5) des Stoffaustauschbodens (4) von der Unterseite (26) des unmittelbar darüber angeordneten Stoffaustauschbodens (4) in einem Bereich von 0 bis 0,3 liegt, insbesondere in einem Bereich von 0 bis 0,1 liegt.

5.  Kolonne (1) nach einem der vorhergehenden Ansprüche ,
    **dadurch gekennzeichnet, dass**
    die Rückführöffnung (14; 14-1 bis 14-3) oberhalb der Ablauföffnung (10) in den Kolonnenhohlraum (3) mündet.

**6.** Kolonne (1) nach einem der Ansprüche 1 bis 4,
**dadurch gekennzeichnet, dass**
die Rückführöffnung (14; 14-1 bis 14-3) auf derselben Höhe wie die Ablauföffnung (10) in den Kolonnenhohlraum (3) mündet.

**7.** Kolonne (1) nach einem der Ansprüche 3 oder 6,
**dadurch gekennzeichnet, dass**
das Verhältnis des vertikalen Abstands des tiefsten Bereichs (L1) der Auffangfläche (5) des Stoffaustauschbodens (4) von der oberen Kante (25) der Rückführöffnung (14; 14-1 bis 14-3) zu dem vertikalen Abstand des tiefsten Bereichs (L1) der Auffangfläche (5) des Stoffaustauschbodens (4) von der Unterseite (26) des unmittelbar darüber angeordneten Stoffaustauschbodens (4) in einem Bereich von 0 bis 0,3 liegt, insbesondere in einem Bereich von 0 bis 0,2 liegt.

**8.** Kolonne (1) nach einem der vorhergehenden Ansprüche,
**dadurch gekennzeichnet, dass**
die Zirkulationsleitung (11) und die Rückführöffnung (14; 14-1 bis 14-3) so angeordnet sind, dass die rückgeführte Flüssigkeit radial in den Kolonnenhohlraum (3) eintritt.

**9.** Kolonne (1) nach einem der vorhergehenden Ansprüche,
**dadurch gekennzeichnet, dass**
mehrere voneinander beabstandete Rückführöffnungen (14-1, 14-2, 14-3) in dem Kolonnenkörper (2) ausgebildet sind, die jeweils in Fluidverbindung mit der Zirkulationsleitung (11) stehen.

**10.** Kolonne (1) nach einem der vorhergehenden Ansprüche,
**dadurch gekennzeichnet, dass**
die oberen Kanten (23) des zylindrischen Körper (8) Überlaufkanten bilden und das Verhältnis des vertikalen Abstands des tiefsten Bereichs (L1) der Auffangfläche (5) des Stoffaustauschbodens (4) von der unteren Kante (24) der Ablauföffnung (10) zu dem vertikalen Abstand des tiefsten Bereichs (L1) der Auffangfläche (5) des Stoffaustauschbodens (4) von der Höhe (L5) der untersten Überlaufkante der zylindrischen Körper (8) in einem Bereich von 0 bis 0,1 liegt, insbesondere in einem Bereich von 0 bis 0,05 liegt.

**11.** Kolonne (1) nach einem der vorhergehenden Ansprüche,
**dadurch gekennzeichnet, dass**
die Rückführöffnungen (14-1, 14-2, 14-3) so relativ zu den zylindrischen Körpern (8) angeordnet sind, dass sich ein mäanderförmiger Flüssigkeitsstrom (19) auf der Auffangfläche (5) des Stoffaustauschbodens (4) ergibt.

**12.** Kolonne (1) nach einem der vorhergehenden Ansprüche,
**dadurch gekennzeichnet, dass**
der Stoffaustauschboden (4) ein Kaminboden ist und der zylindrische Körper (8) ein Kaminkörper ist.

**13.** Thermisches Trennverfahren zwischen wenigstens einem in einer Kolonne (1) nach einem der Ansprüche 1 bis 12 aufsteigenden Gas und wenigstens einer in der Kolonne (1) absteigenden Flüssigkeit.

**14.** Verfahren nach Anspruch 13,
**dadurch gekennzeichnet, dass**
über die Rückführöffnung (14; 14-1 bis 14-3) Flüssigkeit unterhalb des Flüssigkeitsspiegels (22) auf der Auffangfläche (5) eingeleitet wird.

**15.** Verfahren nach Anspruch 13 oder 14,
**dadurch gekennzeichnet, dass**
das aufsteigende Gas und/oder die absteigende Flüssigkeit (Meth)acrylmonomere enthält.

**Claims**

**1.** A column (1) for thermal treatment of a fluid, having a cylindrical, vertical column body (2) which forms a column cavity (3), and a mass transfer tray (4) which is disposed in the column cavity (3) and forms a collecting area (5), and a circulation device (9) having at least one drain orifice (10) formed in the column body (2) above the collecting area (5), a circulation line (11) in fluid connection with the drain orifice (10) and at least one recycling orifice (14; 14-1 to 14-3) which is in fluid connection with the circulation line (11) and is formed in the column body (2) above the collecting area (5), wherein

the mass transfer tray (4) has passage orifices (20) for gas ascending from the bottom, and cylindrical bodies (8) extend upward in the passage orifices (20), where

the upper edges (23) of the cylindrical body (8) form overflow edges and the ratio of the vertical distance of the lowest region (L1) of the collecting area (5) of the mass transfer tray (4) from the upper edge (25) of the recycling orifice (14; 14-1 to 14-3) to the vertical distance of the lowest region (L1) of the collecting area (5) of the mass transfer tray (4) from the height (L5) of the lowermost overflow edge of the cylindrical bodies (8) is within a range from 0 to 0.9, especially within a range from 0 to 0.3.

2. The column (1) according to claim 1,
wherein
the lower edge (24) of the drain orifice (10) is disposed immediately above the lowermost region of the collecting area (5) of the mass transfer tray (4).

3. The column (1) according to claim 1 or 2,
wherein several vertically spaced-apart mass transfer trays (4) are disposed in the column cavity (3) and the drain orifice (10) and the recycling orifice (14; 14-1 to 14-3) are arranged vertically between two adjacent vertically spaced-apart mass transfer trays (4).

4. The column (1) according to claim 3,
wherein
the ratio of the vertical distance of the lowest region (L1) of the collecting area (5) of the mass transfer tray (4) from the lower edge (24) of the drain orifice (10) to the vertical distance of the lowest region (L1) of the collecting area (5) of the mass transfer tray (4) from the underside (26) of the mass transfer tray (4) disposed immediately above it is within a range from 0 to 0.3, especially within a range from 0 to 0.1.

5. The column (1) according to any of the preceding claims,
wherein
the recycling orifice (14; 14-1 to 14-3) opens into the column cavity (3) above the drain orifice (10).

6. The column (1) according to any of claims 1 to 4, wherein
the recycling orifice (14; 14-1 to 14-3) opens into the column cavity (3) at the same height as the drain orifice (10).

7. The column (1) according to either of claims 3 and 6,
wherein
the ratio of the vertical distance of the lowest region (L1) of the collecting area (5) of the mass transfer tray (4) from the upper edge (25) of the recycling orifice (14; 14-1 to 14-3) to the vertical distance of the lowest region (L1) of the collecting area (5) of the mass transfer tray (4) from the underside (26) of the mass transfer tray (4) disposed immediately above it is within a range from 0 to 0.3, especially within a range from 0 to 0.2.

8. The column (1) according to any of the preceding claims,
wherein
the circulation line (11) and the recycling orifice (14; 14-1 to 14-3) are arranged such that the liquid recycled enters the column cavity (3) radially.

9. The column (1) according to any of the preceding claims,
wherein
a plurality of spaced-apart recycling orifices (14-1, 14-2, 14-3) are formed in the column body (2), each of which is in fluid connection with the circulation line (11) this.

10. The column (1) according to any of the preceding claims,
wherein
the upper edges (23) of the cylindrical body (8) form overflow edges and the ratio of the vertical distance of the lowest region (L1) of the collecting area (5) of the mass transfer tray (4) from the lower edge (24) of the drain orifice (10) to the vertical distance of the lowest region (L1) of the collecting area (5) of the mass transfer tray (4) from the height (L5) of the lowermost overflow edge of the cylindrical bodies (8) is within a range from 0 to 0.1, especially within a range from 0 to 0.05.

11. The column (1) according to any of the preceding claims,
wherein
the recycling orifices (14-1, 14-2, 14-3) are arranged relative to the cylindrical bodies (8) so as to result in a meandering liquid flow (19) on the collecting area (5) of the mass transfer tray (4).

12. The column (1) according to any of the preceding claims,
wherein
the mass transfer tray (4) is a chimney tray and the cylindrical body (8) is a chimney body.

13. A thermal separation process between at least one gas ascending within a column (1) according to any of claims 1 to 12 and at least one liquid descending within the column (1).

14. The process according to claim 13,
wherein
liquid is introduced via the recycling orifice (14; 14-1 to 14-3) below the liquid level (22) on the collecting surface (5).

15. The process according to claim 13 or 14,
wherein
the ascending gas and/or the descending liquid comprises (meth)acrylic monomers.


**Revendications**

1. Colonne (1) pour le traitement thermique d'un fluide, comprenant un corps de colonne cylindrique orienté verticalement (2), qui forme une cavité de colonne (3), et un plateau d'échange de matière (4) agencé dans la cavité de colonne (3), qui forme une surface de collecte (5), et
un dispositif de circulation (9), comprenant au moins une ouverture d'évacuation (10) formée dans le corps de colonne (2) au-dessus de la surface de collecte (5), une conduite de circulation (11) en connexion fluidique avec l'ouverture d'évacuation (10), et au moins une ouverture de recyclage (14 ; 14-1 à 14-3) en connexion fluidique avec la conduite de circulation (11), formée dans le corps de colonne (2) au-dessus de la surface de collecte (5),
**caractérisée en ce que**
le plateau d'échange de matière (4) comprend des ouvertures de passage (20) pour un gaz ascendant depuis le bas, et des corps cylindriques (8) s'étendent vers le haut au niveau des ouvertures de passage (20),
les bords supérieurs (23) du corps cylindrique (8) formant des bords de débordement, et le rapport entre l'écart vertical entre la zone la plus basse (L1) de la surface de collecte (5) du plateau d'échange de matière (4) et le bord supérieur (25) de l'ouverture de recyclage (14 ; 14-1 à 14-3) et l'écart vertical entre la zone la plus basse (L1) de la surface de collecte (5) du plateau d'échange de matière (4) et la hauteur (L5) du bord de débordement le plus inférieur du corps cylindrique (8) se situant dans une plage allant de 0 à 0,9, notamment dans une plage allant de 0 à 0,3.

2. Colonne (1) selon la revendication 1, **caractérisée en ce que** le bord inférieur (24) de l'ouverture d'évacuation (10) est agencé directement au-dessus de la zone la plus basse de la surface de collecte (5) du plateau d'échange de matière (4).

3. Colonne (1) selon la revendication 1 ou 2, **caractérisée en ce que** plusieurs plateaux d'échange de matière espacés verticalement (4) sont agencés dans la cavité de colonne (3), et l'ouverture d'évacuation (10) et l'ouverture de recyclage (14 ; 14-1 à 14-3) sont agencées verticalement entre deux plateaux d'échange de matière espacés verticalement voisins (4).

4. Colonne (1) selon la revendication 3, **caractérisée en ce que** le rapport entre l'écart vertical entre la zone la plus basse (L1) de la surface de collecte (5) du plateau d'échange de matière (4) et le bord inférieur (24) de l'ouverture d'évacuation (10) et l'écart vertical entre la zone la plus basse (L1) de la surface de collecte (5) du plateau d'échange de matière (4) et le côté inférieur (26) du plateau d'échange de matière agencé directement au-dessus (4) se situe dans une plage allant de 0 à 0,3, notamment dans une plage allant de 0 à 0,1.

5. Colonne (1) selon l'une quelconque des revendications précédentes, **caractérisée en ce que** l'ouverture de recyclage (14 ; 14-1 à 14-3) débouche au-dessus de l'ouverture d'évacuation (10) dans la cavité de colonne (3).

6. Colonne (1) selon l'une quelconque des revendications 1 à 4, **caractérisée en ce que** l'ouverture de recyclage (14 ; 14-1 à 14-3) débouche à la même hauteur que l'ouverture d'évacuation (10) dans la cavité de colonne (3).

7. Colonne (1) selon l'une quelconque des revendications 3 ou 6, **caractérisée en ce que** le rapport entre l'écart vertical entre la zone la plus basse (L1) de la surface de collecte (5) du plateau d'échange de matière (4) et le bord supérieur (25) de l'ouverture de recyclage (14 ; 14-1 à 14-3) et l'écart vertical entre la zone la plus basse (L1) de la surface de collecte (5) du plateau d'échange de matière (4) et le côté inférieur (26) du plateau d'échange de matière agencé directement au-dessus (4) se situe dans une plage allant de 0 à 0,3, notamment dans une plage allant de 0 à 0,2.

8. Colonne (1) selon l'une quelconque des revendications précédentes, **caractérisée en ce que** la conduite de circulation (11) et l'ouverture de recyclage (14 ; 14-1 à 14-3) sont agencées de telle sorte que le liquide recyclé entre radialement dans la cavité de colonne (3).

9. Colonne (1) selon l'une quelconque des revendications précédentes, **caractérisée en ce que** plusieurs ouvertures de recyclage espacées les unes des autres (14-1, 14-2, 14-3) sont formées dans le corps de colonne (2), et sont chacune en connexion fluidique avec la conduite de circulation (11).

10. Colonne (1) selon l'une quelconque des revendications précédentes, **caractérisée en ce que** les bords supérieurs (23) du corps cylindrique (8) forment des bords de débordement, et le rapport entre l'écart vertical entre la zone la plus basse (L1) de la surface de collecte (5) du plateau d'échange de matière (4) et le bord inférieur (24) de l'ouverture d'évacuation (10) et l'écart vertical entre la zone la plus basse (L1) de la surface de collecte (5) du plateau d'échange de matière (4) et la hauteur (L5) du bord de débordement le plus inférieur du corps cylindrique (8) se situe dans une plage allant de 0 à 0,1, notamment dans une plage allant de 0 à 0,05.

11. Colonne (1) selon l'une quelconque des revendications précédentes, **caractérisée en ce que** les ouvertures de recyclage (14-1, 14-2, 14-3) sont agencées par rapport aux corps cylindriques (8) de telle sorte qu'un écoulement de fluide en méandres (19) soit obtenu sur la surface de collecte (5) du plateau d'échange de matière (4).

12. Colonne (1) selon l'une quelconque des revendications précédentes, **caractérisée en ce que** le plateau d'échange de matière (4) est un plateau à cheminées et le corps cylindrique (8) est un corps à cheminée.

13. Procédé de séparation thermique entre au moins un gaz ascendant dans une colonne (1) selon l'une quelconque des revendications 1 à 12 et au moins un liquide descendant dans la colonne (1).

14. Procédé selon la revendication 13, **caractérisé en ce que** du liquide est introduit par l'ouverture de recyclage (14 ; 14-1 à 14-3) en dessous du niveau du liquide (22) sur la surface de collecte (5).

15. Procédé selon la revendication 13 ou 14, **caractérisé en ce que** le gaz ascendant et/ou le liquide descendant contiennent des monomères (méth)acryliques.

# FIG.1

# FIG.2

# FIG.3

L6

4

26

2

22

L5

23

L4

8

25

L3

14

10

L2

5

21

24

L1

4

# FIG.4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19924532 A1
- DE 10243625 A1
- WO 2008090190 A1
- DE 10332758 A1
- DE 10336386 A1
- DE 19924533 A1
- DE 102010001228 A1
- WO 2004035514 A1
- EP 1125912 A2
- EP 982289 A2
- EP 982287 A1
- DE 10218419 A1
- DE 10230219 A1
- EP 1279429 A1
- US 3988213 A
- EP 1029573 A1
- DE 10156988 A1
- DE 10159823 A1
- EP 0882481 A1
- DE 10257915 A1
- EP 1704906 A1
- DD 279822 A1
- DD 216633 A1
- EP 2460577 A1
- CA 2017980 A1
- US 2141829 A
- EP 00009545 A
- EP 700714 A1
- EP 700893 A1
- DE 19740253 A1
- DE 19740252 A1

**In der Beschreibung aufgeführte Nicht-Patentliteratur**

- Grundlagen der Dimensionierung von Kolonnenböden. Technische Fortschrittsberichte. Verlag Theodor Steinkopf, 1967, vol. 61, 198-211
- *Chemie-Ing.-Techn.,* 1973, vol. 45, 617-620
- *Chemie-Ing. Techn.,* 1973, vol. 45, 617-620
- *Chemie-Ing. Techn.,* 1973, vol. 45, 618
- Grundlagen der Dimensionierung von Kolonnenböden. *Technische Fortschrittsberichte,* vol. 61, 96-138
- *Chem.-Ing.Tech.,* 1986, vol. 58 (1), 19-31
- *Technischen Rundschau Sulzer,* 1979, vol. 2, 49 ff